# EUROPEAN PATENT APPLICATION

(11) **EP 4 729 923 A1**
(43) Date of publication of application: **22.04.2026**
(21) Application number: 25209671.4
(22) Date of filing: 20.10.2025
(51) Int. Cl.: G01N 15/1429, G01N 15/14, G01N 33/49

(54) **SAMPLE MEASUREMENT APPARATUS**

(30) Priority: 21.10.2024 JP 2024185419
(71) Applicant: SYSMEX CORPORATION, Kobe-shi Hyogo 651-0073 (JP)
(72) Inventor: Toya, Yuji, Kobe-shi, Hyogo, 651-0073 (JP); Fujimaki, Kenichi, Kobe-shi, Hyogo, 651-0073 (JP); Tsuji, Tomohiro, Kobe-shi, Hyogo, 651-0073 (JP); Matsuba, Hiroaki, Kobe-shi, Hyogo, 651-0073 (JP); Yamauchi, Masaya, Kobe-shi, Hyogo, 651-0073 (JP)
(74) Representative: Hoffmann Eitle

(57) **Abstract**

Disclosed is a sample measurement apparatus configured to measure a sample collected from a subject, comprising:
a measurement unit configured to execute a measurement of the sample, the measurement including preparing a measurement sample from the sample and a reagent and detecting at least optical signals corresponding to cells in the measurement sample; and
an analysis unit configured to analyze the cells at least according to the optical signals detected in the measurement of the sample by the measurement unit,
wherein the measurement unit is operable to selectively perform a plurality of measurement operations including:
(1) a first measurement operation that includes the measurement of the sample using a first reagent containing a first fluorescent dye that stains leukocytes for classification of leukocytes;
(2) a second measurement operation that includes the measurement of the sample using a second reagent containing a second fluorescent dye that stains cells suspected of malaria infection; and
(3) a third measurement operation that includes the measurement of the sample using the first reagent and the second reagent.

## Description

### CROSS REFERENCE TO RELATED APPLICATIONS

This application claims priority from prior Japanese Patent Application No. 2024-185419, filed on October 21, 2024, entitled "SAMPLE MEASURING APPARATUS", the entire content of which is incorporated herein by reference.

### FIELD OF THE INVENTION

The present invention relates to a sample measurement apparatus for measuring a sample.

### BACKGROUND OF THE INVENTION

A sample analyzer that detects blood cells such as leukocytes and malaria-infected red blood cells is known. For example, WO 2022/115982 A1 describes an analyzer that obtains a scattered light signal, a first fluorescent signal corresponding to a first fluorescent dye that can stain leukocytes, and a second fluorescent signal corresponding to a second fluorescent dye that can stain infected red blood cells, from a measurement sample prepared by mixing a blood sample, a hemolytic reagent, the first fluorescent dye, and the second fluorescent dye, and obtains leukocyte optical information and infected red blood cell optical information in a single test. This analyzer can detect leukocyte parameters and infected red blood cell parameters simultaneously in a single test. This reduces the amount of blood used for the test and lowers the test cost, compared to a case where two measurement samples are prepared to detect each parameter.

### SUMMARY OF THE INVENTION

However, for a sample from a subject who is not suspected of malaria infection, a malaria infection test may be unnecessary. In such a case, there was a problem with the above-mentioned analyzer that the efficiency of sample testing work was reduced, for example, by wasting reagents and time.

In view of such problems, an object of the present invention is to provide a sample measurement apparatus that can improve the efficiency of sample testing work, including tests for malaria infection.

The present invention relates to A sample measurement apparatus (1) configured to measure a sample collected from a subject, comprising:
a measurement unit (10) configured to execute a measurement of the sample, the measurement including preparing a measurement sample from the sample and a reagent and detecting at least optical signals corresponding to cells in the measurement sample; and
an analysis unit (30) configured to analyze the cells at least according to the optical signals detected in the measurement of the sample by the measurement unit (10),
wherein the measurement unit (10) is operable to selectively perform a plurality of measurement operations including:
   (1) a first measurement operation that includes the measurement of the sample using a first reagent containing a first fluorescent dye that stains leukocytes for classification of leukocytes;
   (2) a second measurement operation that includes the measurement of the sample using a second reagent containing a second fluorescent dye that stains cells suspected of malaria infection; and
   (3) a third measurement operation that includes the measurement of the sample using the first reagent and the second reagent.

According to the sample measurement apparatus of the present invention, either or both of the measurement for classifying leukocytes and the measurement of malaria-infected red blood cells can be appropriately selected and executed, for example, according to a measurement order, an analysis result, a medical history, a test result of another apparatus, a travel history to a malaria-endemic area, a season such as a rainy season when malaria is likely to be prevalent, etc. This makes it possible to improve the efficiency of sample testing work, including tests for malaria infection.

According to the present invention, it is possible to improve the efficiency of sample testing work, including tests for malaria infection.

### BRIEF DESCRIPTION OF THE DRAWINGS

FIG. 1 is a perspective view showing the configuration of a sample measurement apparatus according to the first embodiment.
FIG. 2 is a block diagram showing the functional configuration of the measurement unit according to the first embodiment.
FIG. 3 is a diagram showing the configuration of the optical detector according to the first embodiment.
FIG. 4 is a diagram showing the configuration of the electrical detector and the hemoglobin detector according to the first embodiment.
FIG. 5 is a diagram showing a configuration for aspirating and dispensing a sample via an aspiration tube according to the first embodiment.
FIG. 6 is a diagram showing the configuration of a fluid circuit connected to the chambers and the optical detector according to the first embodiment.
FIG. 7 is a diagram showing the configuration of a fluid circuit connected to the chambers, the electrical detector, and the hemoglobin detector according to the first embodiment.
FIG. 8 is a block diagram showing the functional configuration of the conveyer and the analysis unit according to the first embodiment.
FIG. 9 is a diagram showing an example of the relationship between discrete, measurement modes, and measurement items according to the first embodiment.
FIG. 10 is a diagram showing the configuration of a menu screen according to the first embodiment.
FIG. 11 is a diagram showing the configuration of a measurement type switching dialog, a manual measurement dialog, and a measurement unit information display area according to the first embodiment.
FIG. 12 is a diagram showing the configuration of a measurement order registration screen according to the first embodiment.
FIG. 13 is a diagram illustrating a measurement order registration screen when a normal mode is selected as a measurement order according to the first embodiment.
FIG. 14 is a diagram illustrating a measurement order registration screen when a malaria mode is selected as a measurement order according to the first embodiment.
FIG. 15 is a diagram illustrating a measurement order registration screen when a multi-mode is selected as a measurement order according to the first embodiment.
FIG. 16 is a diagram showing the configuration of scattergrams WDF and WNR according to the first embodiment.
FIG. 17 is a diagram showing the configuration of scattergrams M and RET according to the first embodiment.
FIG. 18 is a diagram showing the configuration of scattergram PLT-F according to the first embodiment.
FIG. 19 is a diagram showing the configuration of an analysis result display screen according to the first embodiment.
FIG. 20 is a diagram illustrating an analysis result display screen when a measurement mode and a discrete are set as shown in FIG. 13 according to the first embodiment.
FIG. 21 is a diagram illustrating an analysis result display screen when a measurement mode and a discrete are set as shown in FIG. 14 according to the first embodiment.
FIG. 22 is a diagram illustrating an analysis result display screen when a measurement mode and a discrete are set as shown in FIG. 15 according to the first embodiment.
FIG. 23 is a flowchart showing a process performed by the controller of the analysis unit according to the first embodiment.
FIG. 24 is a diagram showing the configuration of a reflex setting screen according to the second embodiment.
FIG. 25 is a flowchart showing a process performed by the controller of the analysis unit according to the second embodiment.
FIG. 26 is a flowchart showing a process performed by the controller of the analysis unit when a measurement operation is selectively executed based on other information regarding the subject according to a modification 1.
FIG. 27 is a diagram showing the configuration of a fluid circuit connected to the chambers and the optical detector according to a modification 2.
FIG. 28 is a diagram showing the configuration of a fluid circuit connected to the chambers and the optical detector according to a modification 3.
FIG. 29 is a diagram showing the configuration of scattergram WDF according to a modification 4.
FIG. 30 is a diagram showing the configuration of scattergram WDF-1 according to a modification 5.

### DETAILED DESCRIPTION

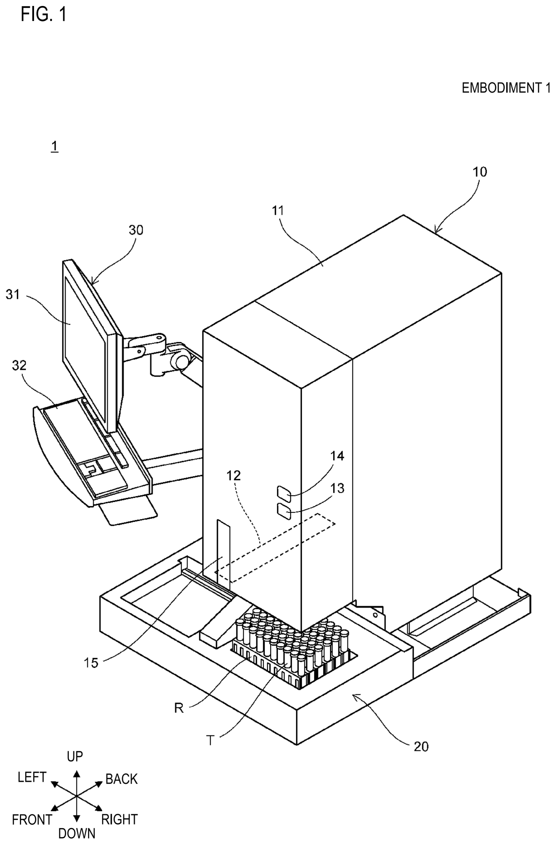

<First Embodiment> FIG. 1 is a perspective view showing the configuration of a sample measurement apparatus 1. FIG. 1 shows the directions of up, down, left, right, front, and back.

The sample measurement apparatus 1 is a blood cell counter that measures leukocytes, red blood cells, malaria-infected red blood cells, platelets, and the like contained in a sample, and performs classification and/or counting of each blood cell. The sample is whole blood collected from a subject. The sample measurement apparatus 1 includes a measurement unit 10, a conveyer 20, and an analysis unit 30.

The conveyer 20 is disposed in front of the measurement unit 10, and conveys a sample rack R holding a plurality of sample containers T to supply it to the measurement unit 10. The measurement unit 10 takes out a sample container T from the sample rack R, sets the taken-out sample container T in a sample setting unit 12, and transfers it to a sample aspiration position inside a housing 11. The measurement unit 10 aspirates a sample from the sample container T at the sample aspiration position, and measures blood cells contained in the sample. The measurement unit 10 returns the sample container T, for which measurement has been completed, to the sample rack R.

A changeover switch 13, a start switch 14, and a cover 15 are provided on the front surface of the housing 11 of the measurement unit 10. When the changeover switch 13 is operated, the cover 15 opens, and the sample setting unit 12 moves to the front of the housing 11. When a sample container T is set in the sample setting unit 12 by an operator and the start switch 14 is operated, the measurement unit 10 drives the sample setting unit 12 to transfer the sample container T to the sample aspiration position and measures the sample in the sample container T. This makes it possible to interrupt the sample containers T on the conveyer 20 and perform a measurement on a predetermined sample container T.

The analysis unit 30 performs analysis including classification and/or counting of blood cells based on a measurement result obtained by the measurement unit 10, and generates an analysis result. The analysis result includes, for example, a result value based on analysis, a graph, a chart, and flag information given to the sample.

The analysis unit 30 includes a display 31 and an operation unit 32. The display 31 displays an analysis result and the like, and includes, for example, a liquid crystal display or an organic EL display. The operation unit 32 receives an operation by an operator, and includes, for example, a mouse or a keyboard. The display 31 and the operation unit 32 may be configured integrally, for example, by a touch-panel type display.

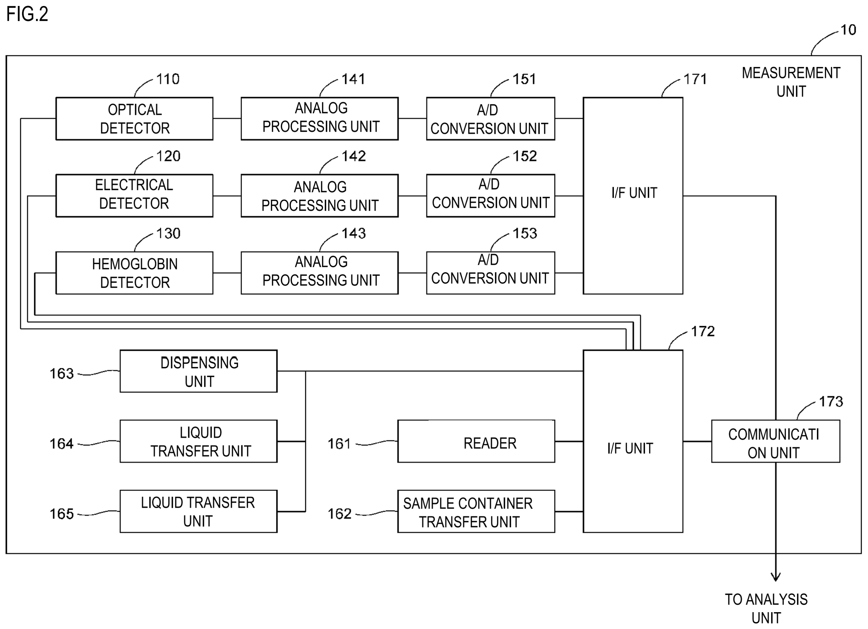

FIG. 2 is a block diagram showing the functional configuration of the measurement unit 10.

The measurement unit 10 includes an optical detector 110, an electrical detector 120, a hemoglobin detector 130, analog processing units 141, 142, and 143, A/D conversion units 151, 152, and 153, a reader 161, a sample container transfer unit 162, a dispensing unit 163, liquid transfer units 164 and 165, IF (interface) units 171 and 172, and a communication unit 173.

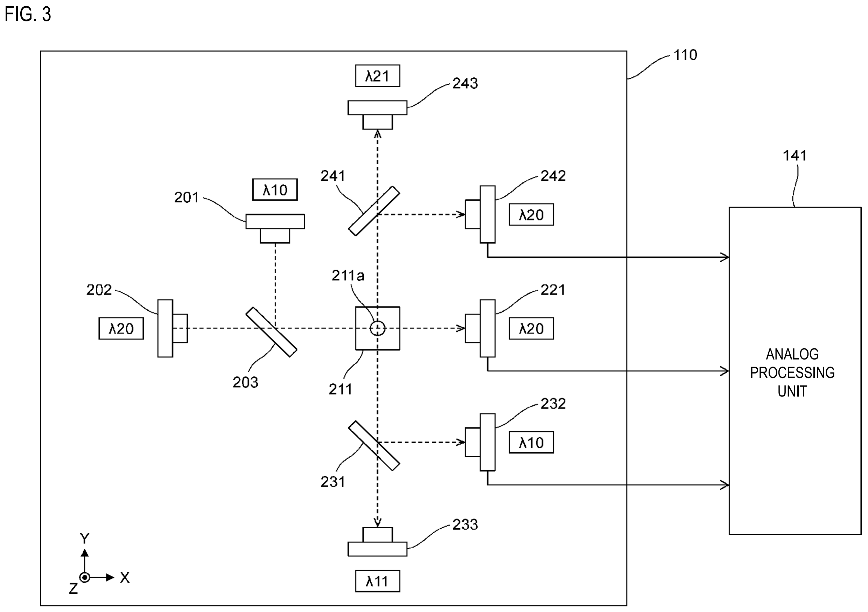

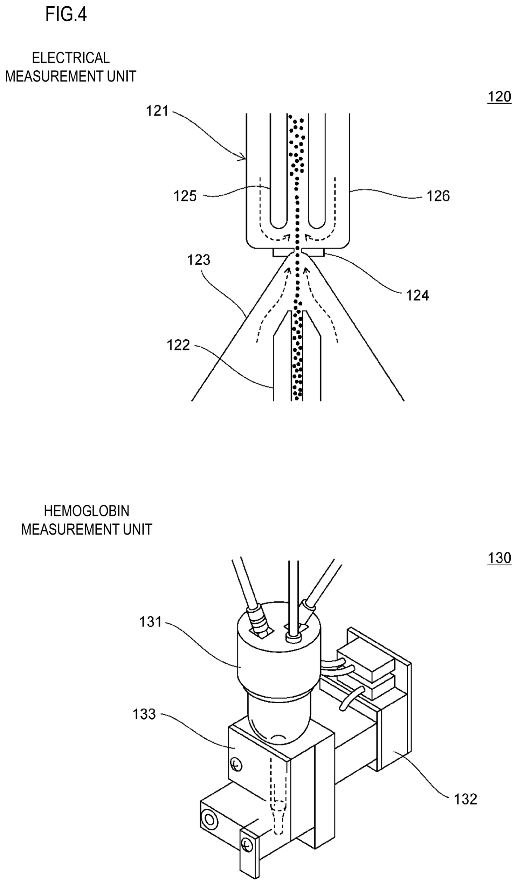

The optical detector 110 detects optical signals corresponding to blood cells in a sample based on a flow cytometry method. The electrical detector 120 detects electrical signals corresponding to blood cells in a sample based on a sheath flow DC detection method. The hemoglobin detector 130 detects optical signals corresponding to the hemoglobin concentration of a sample based on an SLS-hemoglobin method. The configurations of the optical detector 110, the electrical detector 120, and the hemoglobin detector 130 will be described later with reference to FIG. 3 and FIG.4, respectively.

The analog processing units 141, 142, and 143 respectively perform processing such as noise removal and smoothing on analog signals detected by the optical detector 110, the electrical detector 120, and the hemoglobin detector 130. The A/D conversion units 151, 152, and 153 respectively convert the analog signals processed by the analog processing units 141, 142, and 143 into digital signals, and transmit them as a measurement result to the analysis unit 30 via the IF unit 171 and the communication unit 173.

The reader 161 includes a mechanism configured to read a sample ID from a barcode label attached to a sample container T transferred into the housing 11. The sample container transfer unit 162 includes a mechanism configured to take out the sample container T from the sample rack R on the conveyer 20, the sample setting unit 12, and a mechanism configured to transfer the sample setting unit 12 back and forth.

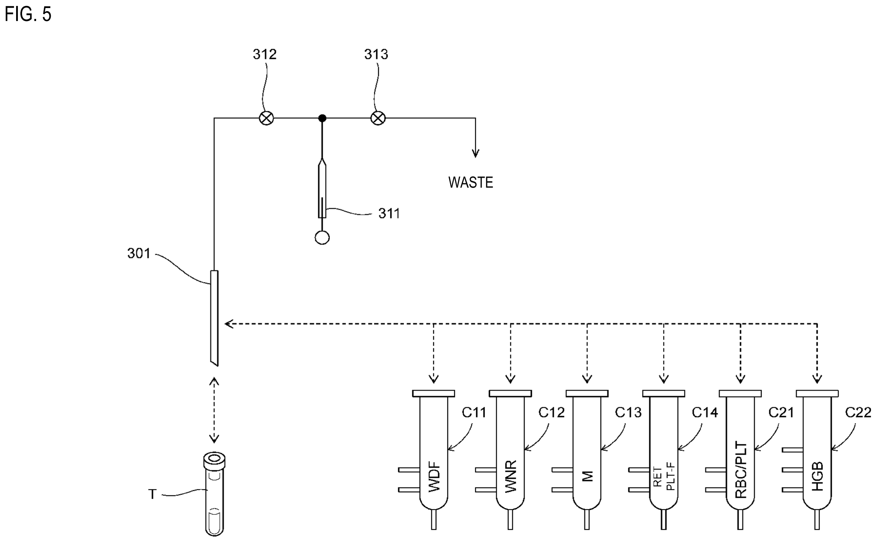

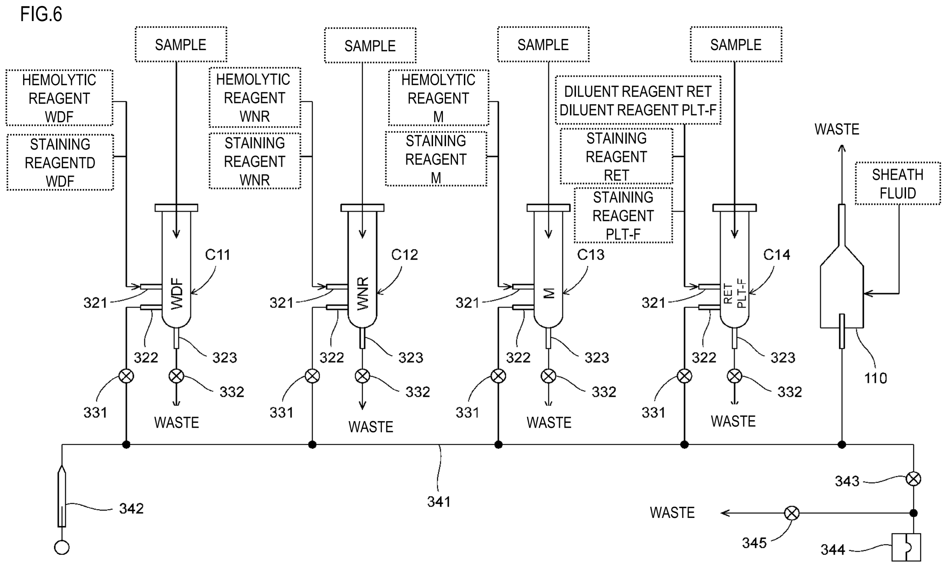

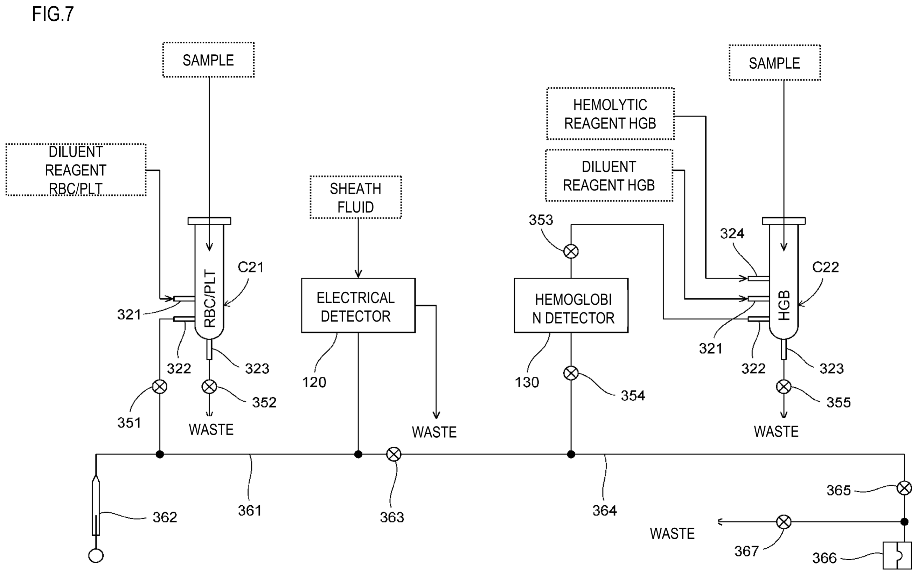

The dispensing unit 163 includes an aspiration tube 301 shown in FIG. 5 and an aspiration tube transfer unit that transfers the aspiration tube 301 in the housing 11. The liquid transfer unit 164 includes a flow path, a syringe pump, and valves shown in FIG. 5, and a mechanism configured to drive the syringe pump and the valves. The liquid transfer unit 165 includes flow paths, chambers C11 to C14, C21, and C22, a syringe pump, a diaphragm pump, and valves shown in Figs. 6 and 7, and a mechanism configured to drive the syringe pump, the diaphragm pump, and the valves.

The communication unit 173 is configured by, for example, a connection terminal based on the USB standard, and performs communication with the analysis unit 30. Each part of the measurement unit 10 is controlled by the analysis unit 30 via the IF units 171, 172 and the communication unit 173.

FIG. 3 is a diagram showing the configuration of the optical detector 110. For convenience, X, Y, and Z axes orthogonal to each other are added to FIG. 3. The Z-axis direction is the flow direction of a measurement sample in a flow cell 211.

The optical detector 110 includes light sources 201 and 202, a dichroic mirror 203, a flow cell 211, a light detector 221, a dichroic mirror 231, light detectors 232 and 233, a dichroic mirror 241, and light detectors 242 and 243.

The light sources 201 and 202 are, for example, semiconductor laser light sources. The light source 201 emits light with a wavelength λ10 in the Y-axis direction, and the light source 202 emits light with a wavelength λ20 in the X-axis direction. The wavelength λ10 is in a bluish-purple wavelength band and is 315 nm or more and 490 nm or less. The wavelength λ20 is in a red wavelength band and is 610 nm or more and 750 nm or less. The dichroic mirror 203 is configured to reflect the light from the light source 201 in the X-axis direction and transmit the light from the light source 202. The dichroic mirror 203 is arranged so that the flow path 211a of the flow cell 211 is irradiated with the light from the light sources 201 and 202 in an overlapping state.

A measurement sample supplied to the optical detector 110 is caused to flow in a flow path 211a of the flow cell 211. When a blood cell in the measurement sample flowing through the flow path 211a is irradiated with light of the wavelength λ10 from the light source 201 and light of the wavelength λ20 from the light source 202, forward scattered light, side scattered light, and fluorescence are generated from a portion of the blood cell irradiated with the light. Here, it is assumed that light of wavelengths λ11 and λ21 are generated when a fluorescent dye that stains blood cells is irradiated with light of wavelengths λ10 and λ20, respectively.

The light detector 221 receives forward scattered light of a wavelength λ20 based on the light from the light source 202 and detects an optical signal corresponding to the received light intensity. The light detector 221 is, for example, a photodiode (PD).

The dichroic mirror 231 is configured to reflect the side scattered light of the wavelength λ10 based on the light from the light source 201 and transmit the fluorescence of the wavelength λ11 based on the light from the light source 201. The light detector 232 receives the side scattered light of the wavelength λ10 and detects an optical signal corresponding to the received light intensity. The light detector 232 is, for example, a photodiode (PD). The light detector 233 receives the fluorescence of the wavelength λ11 and detects an optical signal corresponding to the received light intensity. The light detector 233 is, for example, a photomultiplier tube (PMT), an avalanche photodiode (APD), or a photodiode (PD).

The dichroic mirror 241 is configured to reflect the side scattered light of the wavelength λ20 based on the light from the light source 202 and transmit the fluorescence of the wavelength λ21 based on the light from the light source 202. The light detector 242 receives the side scattered light of the wavelength λ20 and detects an optical signal corresponding to the received light intensity. The light detector 242 is, for example, a photodiode (PD). The light detector 243 receives the fluorescence of the wavelength λ21 and detects an optical signal corresponding to the received light intensity. The light detector 243 is, for example, a photomultiplier tube (PMT), an avalanche photodiode (APD), or a photodiode (PD).

FIG. 4 is a diagram showing the configuration of the electrical detector 120 and the hemoglobin detector 130.

As shown in the upper part of FIG. 4, a flow cell 121 of the electrical detector 120 includes a sample nozzle 122, a chamber 123, an aperture 124, a collection tube 125, and a chamber 126.

A sample nozzle 122 sends a measurement sample supplied to the electrical detector 120 upward. A chamber 123 has a tapered shape that becomes narrower toward the top. A sheath fluid is supplied into the chamber 123. The measurement sample, in a state of being wrapped in the sheath fluid, passes through the aperture 124 and proceeds to a collection tube 125. Blood cells contained in the measurement sample pass through the aperture 124 in a single file. An electrode is provided in the aperture 124. A direct current is supplied between the electrodes of the aperture 124, and an electrical signal corresponding to a change in DC resistance when the measurement sample passes through the aperture 124 is detected. The electrical signal reflects information on the blood cells passing through the aperture 124.

A sheath fluid is supplied to a chamber 126 so as to flow downward in an outer region of the collection tube 125. The sheath fluid flowing outside the collection tube 125 flows into the collection tube 125 after reaching the lower end of the chamber 126. This prevents the blood cells that have passed through the aperture 124 from returning to the aperture 124, and prevents erroneous detection of the blood cells.

As shown in the lower part of FIG. 4, the hemoglobin detector 130 includes a cell 131, a light source unit 132, and a light detector 133.

A cell 131 is made of a translucent material and accommodates a measurement sample supplied to the hemoglobin detector 130. A light source unit 132 irradiates the cell 131 with light of a wavelength having a high absorbance by SLS-hemoglobin. A light detector 133 is arranged opposite to the light source unit 132 with the cell 131 interposed therebetween. The light detector 133 receives transmitted light from the light source unit 132 that has not been absorbed by the measurement sample, and detects an optical signal corresponding to the intensity of the transmitted light. This signal corresponds to absorbance.

FIG. 5 is a diagram showing a configuration for aspirating and dispensing a sample via an aspiration tube 301.

An aspiration tube 301 is transferred by an aspiration tube transfer unit and inserted into the sample container T and the chambers C11 to C14, C21, and C22. An upper end of the aspiration tube 301 is connected to a syringe pump 311 via a flow path, and a valve 312 is disposed in the flow path. The syringe pump 311 includes a piston and a motor, and is configured to aspirate a predetermined amount of a sample from the sample container T via the aspiration tube 301 by applying a predetermined pressure to the flow path, and to dispense the aspirated sample via the aspiration tube 301 by a predetermined amount.

The sample aspirated by the aspiration tube 301 is dispensed into at least one of the chambers C11 to C14, C21, and C22 based on a measurement order set for the sample. In each of the chambers C11 to C14, C21, and C22, the sample and a predetermined liquid reagent are mixed to prepare a measurement sample. When the dispensing of the sample is completed, the syringe pump 311 draws in the sample remaining in the aspiration tube 301 and discards it via a valve 313.

FIG. 6 is a diagram showing the configuration of a fluid circuit connected to the chambers C11 to C14 and the optical detector 110.

The chambers C11 to C14 have the same configuration. The chambers C11 to C14 may have different configurations. The chambers C11 to C14 are containers with open top ends. The sample aspirated from the sample container T by the aspiration tube 301 is dispensed into the chambers C11 to C14 from the openings at the top ends. Each of the chambers C11 to C14 includes an inlet 321 to which a reagent is supplied, an outlet 322 from which a measurement sample prepared in the chamber is discharged, and a waste port 323 from which a liquid in the chamber is discarded. The chambers C11 to C14 are connected to a common optical detector 110 by a common flow path 341.

In a chamber C11, a hemolytic reagent WDF and a staining reagent WDF are supplied via an inlet 321. In the chamber C11, the sample, the hemolytic reagent WDF, and the staining reagent WDF are mixed to prepare a measurement sample WDF. The hemolytic reagent WDF is a reagent that lyses red blood cells and damages the cell membranes of leukocytes to an extent that a fluorescent dye can pass through. The hemolytic reagent WDF is, for example, Lysercell^{®} WDFII (manufactured by Sysmex Corporation). The staining reagent WDF contains a fluorescent dye that stains leukocytes for classification of the leukocytes. The fluorescent dye contained in the staining reagent WDF is, for example, a cyanine fluorescent dye that can be excited by light of a wavelength λ20 and can bind to a nucleic acid. The staining reagent WDF is, for example, Fluorocell^{®} WDF (manufactured by Sysmex Corporation). The measurement sample WDF is supplied to the optical detector 110. The measurement sample WDF is used to classify leukocytes. In this specification, classifying leukocytes means classifying leukocytes into two or more subpopulations.

The hemolytic reagent WDF is not particularly limited and includes, for example, a nonionic surfactant represented by the following formula (I). In formula (I), R₁ is an alkyl group, an alkenyl group, or an alkynyl group having 8 to 25 carbon atoms, and R₂ is an oxygen atom, (COO), or is represented by the following formula (II). [0043]

R₁-R₂-(CH₂CH₂O)n-H (I)

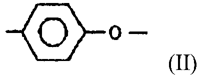

In the hemolytic reagent WDF, the solvent is not particularly limited as long as it can dissolve the nonionic surfactant represented by the formula (I). Examples include water, organic solvents, and mixtures thereof. Examples of the organic solvent include alcohols having 1 to 6 carbon atoms, ethylene glycol, diethylene glycol, polyethylene glycol, and dimethyl sulfoxide (DMSO).

The hemolytic reagent WDF may contain a buffer substance for keeping the pH constant. Examples of the buffer substance include inorganic acid salts, organic acid salts, Good's buffers, and combinations thereof. Examples of the inorganic acid salts include phosphate, borate, and combinations thereof. Examples of the organic acid salts include citrate, malate, and combinations thereof. Examples of Good's buffers include MES, Bis-Tris, ADA, PIPES, Bis-Tris-Propane, ACES, MOPS, MOPSO, BES, TES, HEPES, HEPPS, Tricine, Tris, Bicine, TAPS, and combinations thereof.

In the hemolytic reagent WDF, as the nonionic surfactant represented by the formula (I), for example, polyoxyethylene alkyl ether, polyoxyethylene sterol, polyoxyethylene castor oil, polyoxyethylene sorbitol fatty acid ester, polyoxyethylene alkylamine, polyoxyethylene polyoxypropylene alkyl ether, or a combination thereof can be used. Among them, it is preferable to contain polyoxyethylene alkyl ether. As the polyoxyethylene alkyl ether, it is preferable to select at least one from the group of polyoxyethylene (23) cetyl ether, polyoxyethylene (25) cetyl ether, and polyoxyethylene (30) cetyl ether. More preferably, it is a combination of polyoxyethylene (23) cetyl ether, polyoxyethylene (25) cetyl ether, and a combination thereof, and further preferably polyoxyethylene (23) cetyl ether. Note that, in the hemolytic reagent WDF, one type of nonionic surfactant may be used, or two or more types may be used. The hemolytic reagent WDF may further contain a cationic surfactant or a nonionic surfactant other than the nonionic surfactant represented by the formula (I).

The fluorescent dye contained in the staining reagent WDF is not particularly limited and can be appropriately selected according to the wavelength of light irradiated from a light source. When the wavelength of the light irradiated from the light source is in a bluish-purple wavelength band, examples include propidium iodide, ethidium bromide, ethidium-acridine heterodimer, ethidium diazide, ethidium homodimer-1, ethidium homodimer-2, ethidium monoazide, trimethylenebis [[3-[[4-[[(3-methylbenzothiazol-3-ium)-2-yl]methylene]-1,4-dihydroquinoline]-1-yl]propyl]dimethylammonium] tetraiodide (TOTO-1), 4-[(3-methylbenzothiazol-2(3H)-ylidene)methyl]-1-[3-(trimethylammonio)propyl]quinolinium diiodide (TO-PRO-1), N,N,N',N'-tetramethyl-N,N'-bis[3-[4-[3-[(3-methylbenzothiazol-3-ium)-2-yl]-2-propenylidene]-1,4-dihydroquinolin-1-yl]propyl]-1,3-propanediammonium tetraiodide (TOTO-3), or 2-[3-[[1-[3-(trimethylammonio)propyl]-1,4-dihydroquinoline]-4-ylidene]-1-propenyl]-3-methylbenzothiazol-3-ium diiodide (TOPRO-3), and combinations thereof.

The fluorescent dye contained in the staining reagent WDF also includes a fluorescent dye represented by the following general formula (III).

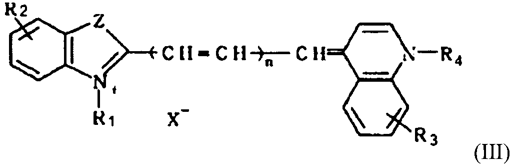

In formula (III), R₁ and R₄ are the same or different, and are a hydrogen atom, an alkyl group, an alkyl chain having a hydroxy group, an alkyl chain having an ether group, an alkyl chain having an ester group, or a benzyl group which may have a substituent. R₂ and R₃ are the same or different, and are a hydrogen atom, a hydroxyl group, a halogen, an alkyl group, an alkenyl group, an alkynyl group, an alkoxy group, an alkylsulfonyl group, or a phenyl group. Z is a sulfur atom, an oxygen atom, or a carbon atom having a methyl group. n is 0, 1, 2, or 3. n is appropriately selected according to the wavelength of light irradiated from a light source. X⁻ is an anion.

In formula (III), the alkyl group may be either a straight chain or a branched chain. When either R₁ or R₄ is an alkyl group having 6 to 18 carbon atoms, the other is preferably a hydrogen atom or an alkyl group having less than 6 carbon atoms. Among the alkyl groups having 6 to 18 carbon atoms, an alkyl group having 6, 8, or 10 carbon atoms is preferable.

In formula (III), examples of the substituent of the benzyl group of R₁ and R₄ include an alkyl group having 1 to 20 carbon atoms, an alkenyl group having 2 to 20 carbon atoms, or an alkynyl group having 2 to 20 carbon atoms. Among them, a methyl group or an ethyl group is particularly preferable.

In formula (III), examples of the alkenyl group of R₂ and R₃ include an alkenyl group having 2 to 20 carbon atoms. Examples of the alkoxy group of R₂ and R₃ include an alkoxy group having 1 to 20 carbon atoms. Among them, a methoxy group or an ethoxy group is particularly preferable.

In formula (III), examples of the anion X⁻ include halogen ions such as F⁻, Cl⁻, Br⁻, and I⁻, CF₃SO₃⁻, and BF₄⁻.

As the hemolytic reagent WDF and the staining reagent WDF, the hemolytic reagent and the staining reagent described in US Patent Application Publication No. 2022/0268763 can be used, and US Patent Application Publication No. 2022/0268763 is incorporated herein by reference.

Returning to FIG. 6, in a chamber C12, a hemolytic reagent WNR and a staining reagent WNR are supplied via an inlet 321. In the chamber C12, the sample, the hemolytic reagent WNR, and the staining reagent WNR are mixed to prepare a measurement sample WNR. The hemolytic reagent WNR is a reagent that lyses red blood cells and damages the cell membranes of leukocytes to an extent that a fluorescent dye can pass through. The hemolytic reagent WNR is, for example, Lysercell^{®} WNR (manufactured by Sysmex Corporation). The staining reagent WNR contains a fluorescent dye that stains leukocytes and nucleated red blood cells for counting leukocytes, basophils, and nucleated red blood cells. The fluorescent dye contained in the staining reagent WNR is, for example, a fluorescent dye that can be excited by light of a wavelength λ20 and can bind to a nucleic acid. The staining reagent WNR is, for example, Fluorocell^{®} WNR (manufactured by Sysmex Corporation). The measurement sample WNR is supplied to the optical detector 110. The measurement sample WNR is used to count leukocytes, basophils, and nucleated red blood cells. In this specification, counting leukocytes means counting the total number of all subpopulations of leukocytes.

In a chamber C13, a hemolytic reagent M and a staining reagent M are supplied via an inlet 321. In the chamber C13, the sample, the hemolytic reagent M, and the staining reagent M are mixed to prepare a measurement sample M. The hemolytic reagent M is a reagent that partially lyses the cell membranes of red blood cells so that a fluorescent dye can pass through while keeping malaria parasites inside the red blood cells. The hemolytic reagent M is, for example, Lysercell^{®} M (manufactured by Sysmex Corporation). The staining reagent M contains a fluorescent dye that stains red blood cells suspected of malaria infection. The fluorescent dye contained in the staining reagent M is, for example, a DNA-selective fluorescent dye that can be excited by light of a wavelength λ10 and stains DNA more strongly than RNA. The staining reagent M is, for example, Fluorocell^{®} M (manufactured by Sysmex Corporation). The measurement sample M is supplied to the optical detector 110. The measurement sample M is used to count malaria-infected red blood cells.

Note that the amount of the sample dispensed into the chamber C13 by the syringe pump 311 (see FIG. 5) is preferably larger than the amount of the sample dispensed into the chamber C11 by the syringe pump 311. Therefore, it is preferable that the operation of the syringe pump 311 for preparing the measurement sample WDF and the operation for preparing the measurement sample M are different from each other. Since the number of malaria-infected red blood cells is often smaller than the number of normal leukocytes, by operating the syringe pump 311 as described above, malaria-infected red blood cells can be accurately counted.

The hemolytic reagent M is not particularly limited, and for example, contains a first surfactant having a predetermined lysing power on the cell membranes of red blood cells and a second surfactant having a lysing power weaker than that of the first surfactant, has a pH of 5 to 7, and has an osmotic pressure of 200 to 300 mOsm/kg H₂O.

As the first surfactant and the second surfactant, any of an anionic surfactant, a nonionic surfactant, and a cationic surfactant can be used, but a cationic surfactant is preferably used.

Specifically, as the cationic surfactant, octyltrimethylammonium bromide (OTAB), decyltrimethylammonium bromide (DTAB), lauryltrimethylammonium chloride (LTAC), myristyltrimethylammonium bromide (MTAB), cetylpyridinium chloride (CPC), stearyltrimethylammonium chloride (STAC), or the like can be used.

The lysing power of a surfactant on a red blood cell membrane mainly depends on the number of carbon atoms contained in the surfactant. Specifically, the larger the number of carbon atoms, the stronger the lysing power becomes in the case of a quaternary ammonium salt as the carbon chain of the straight-chain alkyl group portion becomes longer, but it is more likely to solidify at room temperature. Therefore, by using a surfactant with a small number of carbon atoms for the red blood cell membrane partial lysing reagent, the solubility of the red blood cell membrane partial lysing reagent in a solvent can be increased, and the influence on the red blood cell membrane can be adjusted. Therefore, when the first surfactant and the second surfactant are quaternary ammonium salts having long-chain alkyl groups, it is preferable that the number of carbon atoms of the long-chain alkyl group of the second surfactant is less than the number of carbon atoms of the long-chain alkyl group of the first surfactant.

Specifically, a combination in which the first surfactant is stearyltrimethylammonium chloride (STAC) and the second surfactant is lauryltrimethylammonium chloride (LTAC) is preferably used.

The combination of the first surfactant and the second surfactant and the respective concentrations of these surfactants in the reagent are specifically combined and selected so that the red blood cell membranes are lysed to an extent that the fluorescent dye can pass into the red blood cells and the shape of the red blood cells can be maintained. For example, in the case of a combination of STAC with 21 carbon atoms (the longest alkyl group has an 18-carbon chain) and LTAC with 15 carbon atoms (the longest alkyl group has a 12-carbon chain), a condition where the STAC is mixed in a range of 40 to 600 ppm and the LTAC is mixed in a range of 500 to 1400 ppm is preferably used.

The hemolytic reagent M can contain a buffer to maintain a pH of 5 to 7. By maintaining such a pH range, the cell membranes of the red blood cells can be partially lysed so that the fluorescent dye can pass through while keeping the malaria parasites inside the red blood cells.

As the buffer, citric acid, phosphoric acid, succinic acid, tricine, or the like can be used. Hydrochloric acid, sodium hydroxide, or the like may be added as a pH adjusting agent to adjust the pH.

Furthermore, the hemolytic reagent M preferably contains an osmotic pressure adjusting agent to maintain the osmotic pressure in the range of 200 to 300 mOsm/kg H₂O. If the osmotic pressure is less than 200 mOsm/kg H₂O, the reagent or the liquid component in the blood tends to be taken into the red blood cells and the red blood cell swells, which may cause hypotonic hemolysis of the red blood cells. If the osmotic pressure exceeds 300 mOsm/kg H₂O, a fluorescent dye to be described later for malaria parasite detection becomes difficult to enter the red blood cells, and a structural change due to the shrinkage of the red blood cells may occur.

As the osmotic pressure adjusting agent, alkali metal halides such as sodium chloride, alkaline earth halides such as magnesium chloride, carboxylic acid metal salts such as propionic acid, and saccharides such as glucose and mannose are preferably used.

Furthermore, the hemolytic reagent M may contain an antiseptic agent such as 2-pyridylthio-1-oxide sodium or β-phenethyl alcohol, if necessary.

Further, the hemolytic reagent M may be diluted with purified water, ethanol, or the like for concentration adjustment within a range that does not affect pH and osmotic pressure.

The hemolytic reagent M preferably further includes a nonionic surfactant that does not substantially lyse the cell membranes of red blood cells. This makes it possible to classify malaria-infected red blood cells more accurately according to the growth stage of the malaria parasite.

Specifically, as the nonionic surfactant, polyoxyethylene sorbitan fatty acid esters such as polyoxyethylene (20) sorbitan monoisostearate and polyoxyethylene (20) sorbitan monooleate; castor oils such as polyoxyethylene (30) hydrogenated castor oil and polyoxyethylene (50) hydrogenated castor oil; polyoxyethylene phytosterols such as polyoxyethylene (20) phytosterol (hereinafter abbreviated as "POE (20) phytosterol") and polyoxyethylene (25) phytostanol (hereinafter abbreviated as "POE (25) phytostanol"); polyoxyethylene alkyl ethers such as polyoxyethylene (21) lauryl ether, polyoxyethylene (16) oleyl ether, and polyoxyethylene (20) oleyl ether; polyoxyethylene/polyoxypropylene alkyl ethers such as polyoxyethylene (20) polyoxypropylene (6) decyltetradecyl ether and polyoxyethylene (20) polyoxypropylene (8) cetyl ether; and polyoxyethylene fatty acid esters such as polyoxyethylene (10) monolaurate are preferably used. The numerical value in parentheses of polyoxyethylene indicates the number of carbon atoms in the polyethylene chain portion.

The fluorescent reagent contained in the staining reagent M is not particularly limited, and is, for example, a DNA-selective fluorescent dye, and preferably a DNA-selective bisbenzimide fluorescent dye. A DNA-selective fluorescent dye is a fluorescent dye that stains DNA more strongly than RNA, and a DNA-selective bisbenzimide fluorescent dye has a bisimide skeleton.

As such a dye, for example, a dye having a structure as shown in the following formula (IV) (for example, Hoechst 34580 manufactured by Invitrogen Corporation) is preferably used.

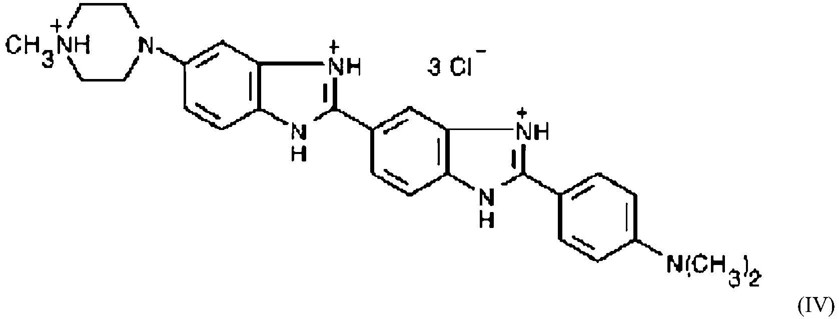

In addition to the above-mentioned dyes, the DNA-selective bisbenzimide fluorescent dyes include Hoechst 33258 and Hoechst 33342. These dyes have different side chains from Hoechst 34580, but can be excited in a bluish-purple wavelength band (315 nm or more and 490 nm or less).

As the hemolytic reagent M and the staining reagent M, the hemolytic reagent and the staining reagent described in US Patent Application Publication No. 2006/0223137 can be used, and US Patent Application Publication No. 2006/0223137 is incorporated herein by reference.

Returning to FIG. 6, in a chamber C14, a diluent reagent RET and a staining reagent RET are supplied via an inlet 321. In the chamber C14, the sample, the diluent reagent RET, and the staining reagent RET are mixed to prepare a measurement sample RET. The diluent reagent RET is a reagent for diluting a sample. The diluent reagent RET is, for example, Cellpack^{®} DFL (manufactured by Sysmex Corporation). The staining reagent RET is a reagent for staining blood cell components. The staining reagent RET is, for example, Fluorocell^{®} RET (manufactured by Sysmex Corporation). The measurement sample RET is supplied to the optical detector 110. The measurement sample RET is used to count reticulocytes.

The chamber C14 is also supplied with a diluent reagent PLT-F and a staining reagent PLT-F via the inlet 321 when the chamber C14 is not used for the preparation of the measurement sample RET. In the chamber C14, the sample, the diluent reagent PLT-F, and the staining reagent PLT-F are mixed to prepare a measurement sample PLT-F. The diluent reagent PLT-F is a reagent for diluting a sample. The diluent reagent PLT-F is, for example, Cellpack^{®} DFL (manufactured by Sysmex Corporation). The staining reagent PLT-F is a reagent for staining blood cell components. The staining reagent PLT-F is, for example, Fluorocell^{®} PLT (manufactured by Sysmex Corporation). The measurement sample PLT-F is supplied to the optical detector 110. The measurement sample PLT-F is used to count platelets.

An outlet 322 of each of the chambers C11 to C14 is connected to a flow path 341 via a valve 331. A syringe pump 342, a valve 343, and the optical detector 110 are connected to the flow path 341. A diaphragm pump 344 is connected to the flow path 341 via the valve 343. A valve 345 is connected to the flow path between the valve 343 and the diaphragm pump 344. A waste port 323 of each of the chambers C11 to C14 is connected to a waste flow path via a valve 332.

When the preparation of the measurement sample is completed in each chamber shown in FIG. 6, the diaphragm pump 344 draws the prepared measurement sample from the corresponding chamber into the flow path 341. A syringe pump 342 supplies the measurement sample stored in the flow path 341 to the optical detector 110. The syringe pump 342 is configured to be able to transfer a predetermined amount of the measurement sample stored in the flow path 341 to the optical detector 110 by applying a predetermined pressure to the flow path 341.

The optical detector 110 causes the measurement sample and a sheath fluid to flow through a flow cell 211 (see FIG. 3) and detects optical signals corresponding to blood cells in the measurement sample based on a flow cytometry method.

Specifically, the optical detector 110 measures a measurement sample WDF and detects optical signals corresponding to leukocytes and the like in the measurement sample WDF. The optical detector 110 measures a measurement sample WNR and detects optical signals corresponding to leukocytes, nucleated red blood cells, and the like in the measurement sample WNR. The optical detector 110 measures a measurement sample M and detects optical signals corresponding to leukocytes, malaria-infected red blood cells, and the like in the measurement sample M. The optical detector 110 measures a measurement sample RET and detects optical signals corresponding to reticulocytes and the like in the measurement sample RET. The optical detector 110 measures a measurement sample PLT-F and detects optical signals corresponding to platelets and the like in the measurement sample PLT-F. The optical detector 110 performs measurement of each measurement sample individually. The measurement samples that have passed through the flow cell 211 of the optical detector 110 are discarded.

The measurement time of the measurement sample M by the optical detector 110 is preferably longer than the measurement time of the measurement sample WDF. The measurement time can be adjusted by changing the amount of measurement sample supplied to the optical detector 110 by the syringe pump 342, and/or by changing the flow rate of the measurement sample supplied to the optical detector 110 by the syringe pump 342. Since the number of red blood cells infected with malaria is often smaller than the number of normal leukocytes, the syringe pump 342 and the optical detector 110 operate as described above to accurately count the red blood cells infected with malaria.

When the measurement of one measurement sample by the optical detector 110 is completed, a cleaning liquid is supplied to the chamber in which the measurement sample was prepared, and the cleaning liquid in the chamber is discarded via the waste port 323 and the valve 332, and is discharged to the flow path 341 via the outlet 322. The cleaning liquid discharged to the flow path 341 is discarded via the valve 345.

FIG. 7 is a diagram showing a configuration of a fluid circuit connected to the chambers C21 and C22, the electrical detector 120, and the hemoglobin detector 130.

The chambers C21 and C22 have the same configuration as the above-described chambers C11 to C14. However, the chamber C22 further includes an inlet 324 to which a reagent is supplied. The sample aspirated from the sample container T by the aspiration tube 301 is discharged into the chambers C21 and C22 from an opening at the upper end thereof.

The diluent reagent RBC/PLT is supplied to the chamber C21 via the inlet 321. In the chamber C21, the sample and the diluent reagent RBC/PLT are mixed to prepare a measurement sample RBC/PLT. The diluent reagent RBC/PLT is a reagent for diluting the sample. The diluent reagent RBC/PLT is, for example, CELLPACK ^{®} DCL (manufactured by Sysmex Corporation). The measurement sample RBC/PLT is supplied to the electrical detector 120. The measurement sample RBC/PLT is used for counting red blood cells and platelets.

A hemolytic reagent HGB is supplied to the chamber C22 via an inlet 324, and a diluent reagent HGB is supplied via an inlet 321. In the chamber C22, the sample, the hemolytic reagent HGB, and the diluent reagent HGB are mixed to prepare a measurement sample HGB. The hemolytic reagent HGB is a reagent for eluting hemoglobin from red blood cells. The hemolytic reagent HGB is, for example, SULFOLYSER ^{®} (manufactured by Sysmex Corporation). The diluent reagent HGB is a reagent for diluting the sample. The diluent reagent HGB is, for example, CELLPACK ^{®} DCL (manufactured by Sysmex Corporation). The measurement sample HGB is supplied to the hemoglobin detector 130. The measurement sample HGB is used for obtaining a hemoglobin concentration.

An outlet 322 of the chamber C21 is connected to a flow path 361 via a valve 351. A syringe pump 362, a valve 363, and the electrical detector 120 are connected to the flow path 361. An outlet 322 of the chamber C22 is connected to the hemoglobin detector 130 via a valve 353. The hemoglobin detector 130 is connected to a flow path 364 via a valve 354. Valves 363 and 365 are connected to the flow path 364. A diaphragm pump 366 is connected to the flow path 364 via a valve 365. A valve 367 is connected to a flow path between the valve 365 and the diaphragm pump 366. Waste ports 323 of the chambers C21 and C22 are respectively connected to a waste flow path via valves 352 and 355.

When the preparation of the measurement sample RBC/PLT is completed in the chamber C21, the diaphragm pump 366 draws the measurement sample RBC/PLT from the chamber C21 into the flow path 361. The syringe pump 362 supplies the measurement sample RBC/PLT stored in the flow path 361 to the electrical detector 120. The syringe pump 362 is configured to be able to transfer a predetermined amount of the measurement sample stored in the flow path 361 to the electrical detector 120 by applying a predetermined pressure to the flow path 361.

The electrical detector 120 causes the measurement sample RBC/PLT and a sheath fluid to flow into a flow cell 121 (see the upper part of FIG. 4) and detects electrical signals corresponding to red blood cells, platelets, etc. in the measurement sample RBC/PLT based on a sheath flow DC detection method. The measurement sample RBC/PLT that has passed through the flow cell 121 of the electrical detector 120 is discarded.

When the measurement of the measurement sample RBC/PLT by the electrical detector 120 is completed, a cleaning liquid is supplied to the chamber C21, and the cleaning liquid in the chamber C21 is discarded via the waste port 323, and is discharged to the flow path 361 via the outlet 322. The cleaning liquid discharged to the flow path 361 is discarded via the valve 367.

When the preparation of the measurement sample HGB in the chamber C22 is completed, the measurement sample HGB is supplied to the hemoglobin detector 130 via the valve 353. The hemoglobin detector 130 detects optical signals corresponding to the hemoglobin concentration based on the measurement sample HGB by an SLS-hemoglobin method. The measurement sample HGB used for the measurement by the hemoglobin detector 130 is discarded.

When the measurement of the measurement sample HGB by the hemoglobin detector 130 is completed, a cleaning liquid is supplied to the chamber C22, and the cleaning liquid in the chamber C22 is discarded via the waste port 323, and is discharged to the hemoglobin detector 130 via the outlet 322. The cleaning liquid discharged to the hemoglobin detector 130 is discharged to the flow path 364 through the inside of the hemoglobin detector 130. The cleaning liquid discharged to the flow path 364 is discarded via the valve 367.

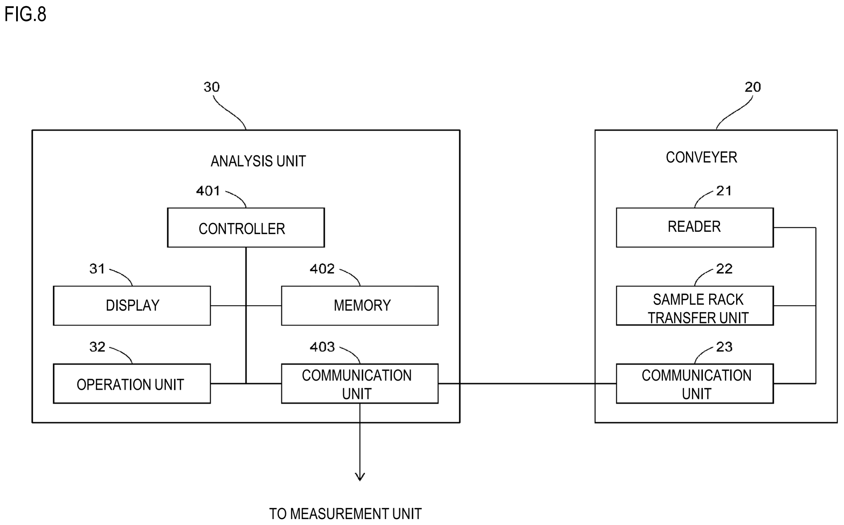

FIG. 8 is a block diagram showing a functional configuration of the conveyer 20 and the analysis unit 30.

The conveyer 20 includes a reader 21, a sample rack transfer unit 22, and a communication part 23.

The reader 21 includes a mechanism configured to read a rack ID and a sample ID from barcode labels attached to the sample rack R and the sample container T, respectively, which are transferred on the conveyer 20. The sample rack transfer unit 22 includes a mechanism configured to transfer the sample rack R on the conveyer 20. The communication part 23 is configured with a connection terminal based on a USB standard and communicates with the analysis unit 30. Each part of the conveyer 20 is controlled by the analysis unit 30 via the communication part 23.

The analysis unit 30 includes a controller 401, a storage 402, and a communication part 403. The analysis unit 30 also includes the display 31 and the operation unit 32 shown in FIG. 1.

The controller 401 includes, for example, a CPU. The controller 401 executes a computer program stored in the storage 402 to analyze the sample and to control the measurement unit 10 and the conveyer 20. The storage 402 includes, for example, an SSD or an HDD. The storage 402 stores measurement results received from the measurement unit 10, analysis results based on the measurement results, and programs for controlling the analysis unit 30, the measurement unit 10, and the conveyer 20.

The communication part 403 is configured with a connection terminal based on a USB standard and communicates with a communication part 173 (see FIG. 2) of the measurement unit 10 and the communication part 23 of the conveyer 20 via a cable based on the USB standard. The controller 401 receives the sample ID read by the reader 161 (see FIG. 2) of the measurement unit 10, the measurement results obtained by the measurement unit 10, and information indicating that the changeover switch 13 and the start switch 14 have been operated, via the communication part 403. In addition, the controller 401 receives the rack ID and the sample ID read by the reader 21 of the conveyer 20 and information indicating that the sample rack R has been set in the conveyer 20, via the communication part 403.

As will be described later, the operator specifies a discrete to which a plurality of measurement items such as CBC, CBC+DIFF, CBC+DIFF+RET, CBC+MI, CBC+DIFF+RET, or MI are associated in advance, and sets a measurement order including measurement contents of the sample. When the controller 401 accepts a measurement order from the operator, it stores the accepted measurement order in the storage 402. When the controller 401 receives a sample ID read by the reader 161 (see FIG. 2) of the measurement unit 10, it reads a measurement order corresponding to the received sample ID from the storage 402 and controls the measurement unit 10 to perform measurement of the sample based on the measurement order.

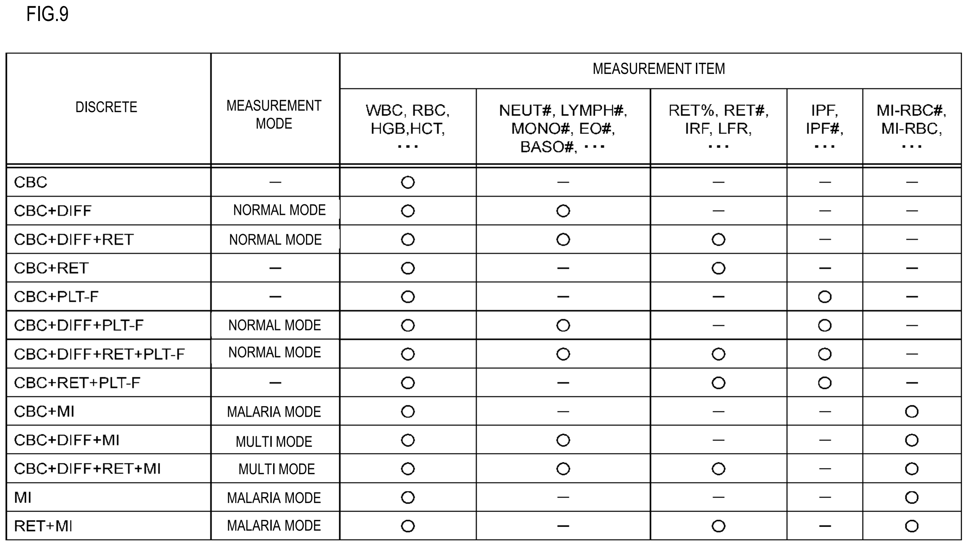

FIG. 9 is a diagram showing an example of a relationship among discretes, measurement modes, and measurement items.

A discrete corresponds to a combination of predetermined measurement items. In FIG. 9, "CBC," "DIFF," "RET," "PLT-F," and "MI" (hereinafter referred to as "sub-discrete") each correspond to predetermined measurement items different from other sub-discrete items, and a discrete is one sub-discrete or a combination of a plurality of sub-discretes.

"CBC" is a sub-discrete that measures the measurement sample RBC/PLT with the electrical detector 120, measures the measurement sample HGB with the hemoglobin detector 130, measures the measurement sample WNR with the optical detector 110, counts red blood cells, platelets, and leukocytes, and obtains a hemoglobin concentration, a hematocrit value (HCT), a mean corpuscular volume (MCV), a mean corpuscular hemoglobin (MCH), a mean corpuscular hemoglobin concentration (MCHC), and the like. In "CBC", since the measurement sample WDF is not prepared, classification of leukocytes is not performed. "DIFF" is a sub-discrete that measures the measurement sample WDF and the measurement sample WNR with the optical detector 110, classifies leukocytes into five subpopulations (neutrophils, lymphocytes, monocytes, eosinophils, and basophils), and counts each of the classified five subpopulations. "RET" is a sub-discrete that measures the measurement sample RET with the optical detector 110 and counts reticulocytes. "PLT-F" is a sub-discrete that measures the measurement sample PLT-F with the optical detector 110 and counts platelets. "MI" is a sub-discrete that measures the measurement sample RBC/PLT with the electrical detector 120, measures the measurement sample M with the optical detector 110, counts red blood cells infected with malaria, and calculates a ratio of the red blood cells infected with malaria to the number of red blood cells. In "MI", the analysis results obtained by measuring the measurement sample RBC/PLT with the electrical detector 120 can be used for the number of red blood cells.

In this embodiment, as shown in FIG. 9, thirteen types of discretes are preset, and measurement items correspond to each of the thirteen types of discretes.

The measurement items corresponding to the sub-discrete "CBC" are, for example, WBC, RBC, HGB, HCT, MCV, MCH, MCHC, PLT, NRBC#, NRBC%, and the like. NRBC# is the number of nucleated red blood cells, and NRBC% is a ratio of the number of nucleated red blood cells to the number of leukocytes. The measurement items corresponding to the sub-discrete "DIFF" are, for example, NEUT#, LYMPH#, MONO#, EO#, BASO#, NEUT%, LYMPH%, MONO%, EO%, BASO%, and the like. NEUT#, LYMPH#, MONO#, EO#, and BASO# are the numbers of neutrophils, lymphocytes, monocytes, eosinophils, and basophils, respectively, and NEUT%, LYMPH%, MONO%, EO%, and BASO% are the ratios of the numbers of neutrophils, lymphocytes, monocytes, eosinophils, and basophils, respectively, to the number of leukocytes. The measurement items corresponding to the sub-discrete "RET" are, for example, RET%, RET#, and the like. RET% is a ratio of the number of reticulocytes to the total number of mature red blood cells and reticulocytes, and RET# is the number of reticulocytes. The measurement items corresponding to the sub-discrete "PLT-F" are, for example, IPF, IPF#, PLT, and the like. IPF is a ratio of the number of immature platelets to the total number of mature platelets and immature platelets, and IPF# is the number of immature platelets. PLT is the total number of mature platelets and immature platelets. The measurement items corresponding to the sub-discrete "MI" are, for example, MI-RBC# and MI-RBC%. MI-RBC% is a ratio of the number of red blood cells infected with malaria to the total number of red blood cells not infected with malaria and red blood cells infected with malaria. MI-RBC# is the number of red blood cells infected with malaria.

When any of the thirteen types of discretes shown in FIG. 9 is specified, the controller 401 measures the corresponding measurement sample, performs analysis on the corresponding measurement items, and obtains an analysis result.

In this embodiment, discretes that perform classification of leukocytes and do not perform classification and counting of red blood cells infected with malaria ("CBC+DIFF," "CBC+DIFF+RET," "CBC+DIFF+PLT-F," "CBC+DIFF+RET+PLT-F") correspond to "Normal Mode" as shown in the measurement mode column of FIG. 9. Discretes that do not perform classification of leukocytes and perform counting of red blood cells infected with malaria ("CBC+MI," "MI," "RET+MI") correspond to "Malaria Mode" as shown in the measurement mode column of FIG. 9. Discretes that perform classification of leukocytes and counting of red blood cells infected with malaria ("CBC+DIFF+MI," "CBC+DIFF+RET+MI") correspond to "Multi Mode" as shown in the measurement mode column of FIG. 9. The discrete items other than those shown in FIG. 9 do not correspond to any of "Normal Mode," "Malaria Mode," and "Multi Mode."

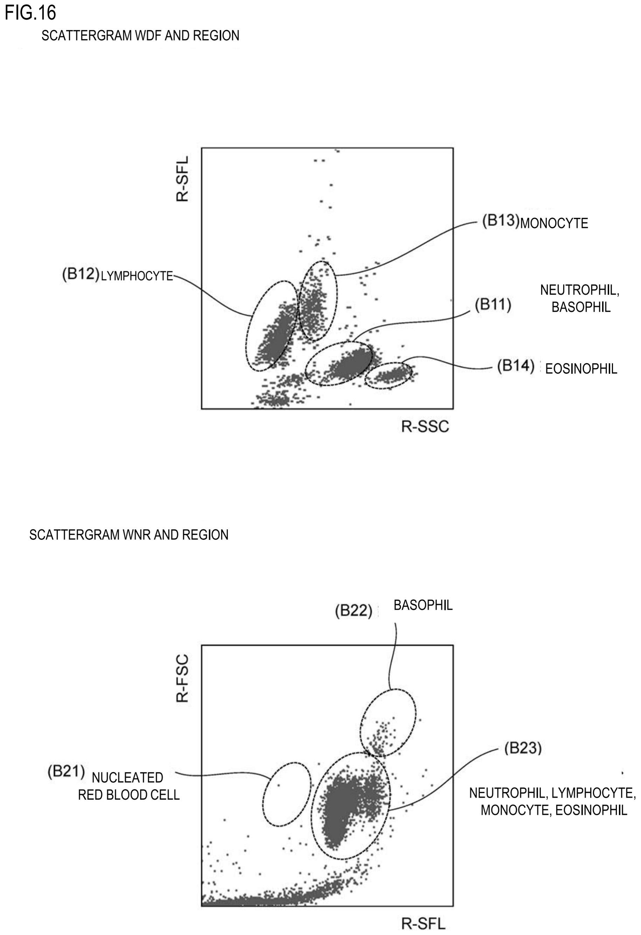

When a discrete corresponding to the "Normal Mode" is selected, the measurement unit 10 executes at least a measurement operation (first measurement operation) for measuring a sample using a staining reagent WDF. Specifically, the measurement unit 10 prepares a measurement sample WDF and detects optical signals corresponding to cells in the measurement sample WDF by the optical detector 110. The analysis unit 30 creates a scattergram WDF shown in the upper part of FIG. 16 based on the measurement results transmitted from the measurement unit 10, and classifies leukocytes into a plurality of subpopulations. Note that the measurement unit 10 does not execute a measurement operation (second measurement operation) for measuring a sample using a staining reagent M for a discrete corresponding to the "Normal Mode."

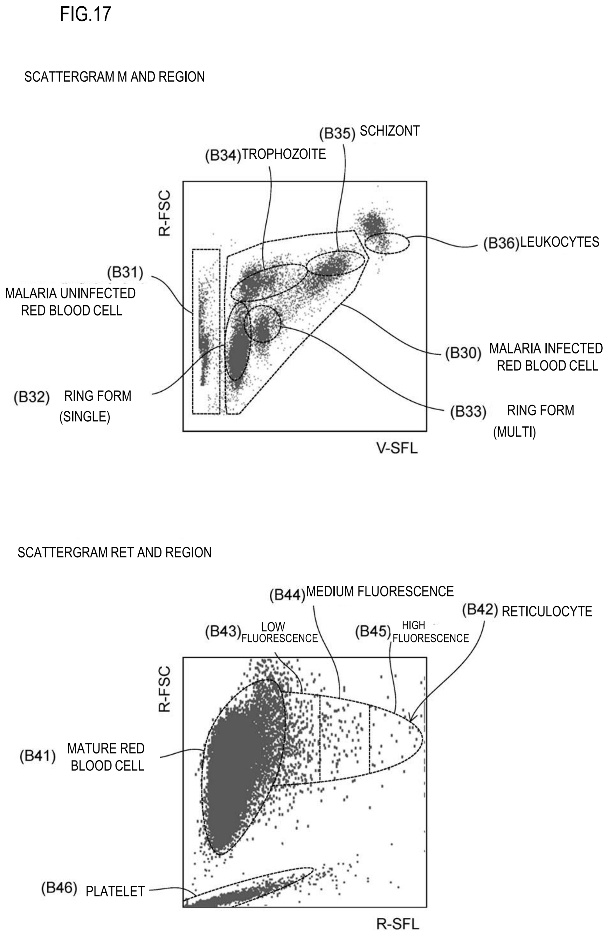

When a discrete corresponding to the "Malaria Mode" is selected, the measurement unit 10 executes at least a measurement operation (second measurement operation) for measuring a sample using a staining reagent M. Specifically, the measurement unit 10 prepares a measurement sample M and detects optical signals corresponding to cells in the measurement sample M by the optical detector 110. The analysis unit 30 creates a scattergram M shown in the upper part of FIG. 17 based on the measurement results transmitted from the measurement unit 10 and counts red blood cells infected with malaria. Note that the measurement unit 10 does not execute a measurement operation (first measurement operation) for measuring a sample using a staining reagent WDF for a discrete corresponding to the "Malaria Mode."

When a discrete corresponding to the "Multi Mode" is selected, the measurement unit 10 executes at least a measurement operation (third measurement operation) for measuring a sample using a staining reagent WDF and a staining reagent M. Specifically, the measurement unit 10 prepares a measurement sample WDF, detects optical signals corresponding to cells in the measurement sample WDF by the optical detector 110, prepares a measurement sample M, and detects optical signals corresponding to cells in the measurement sample M by the optical detector 110. That is, when a discrete corresponding to the "Multi Mode" is selected, the measurement unit 10 executes at least the first measurement operation and the second measurement operation. The analysis unit 30 creates a scattergram WDF shown in the upper part of FIG. 16 based on the measurement results transmitted from the measurement unit 10, classifies leukocytes, creates a scattergram M shown in the upper part of FIG. 17, and counts red blood cells infected with malaria.

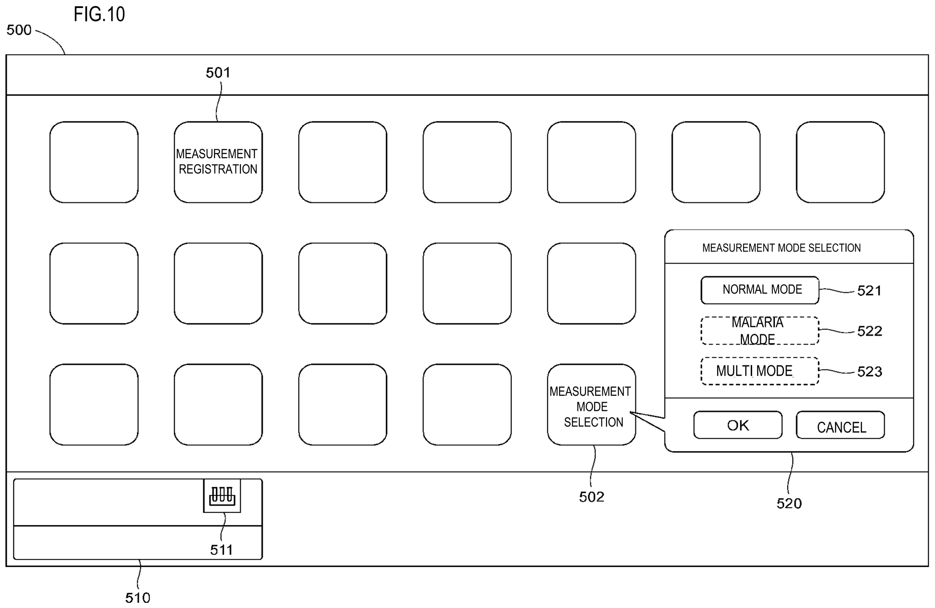

FIG. 10 is a diagram showing a configuration of a menu screen 500.

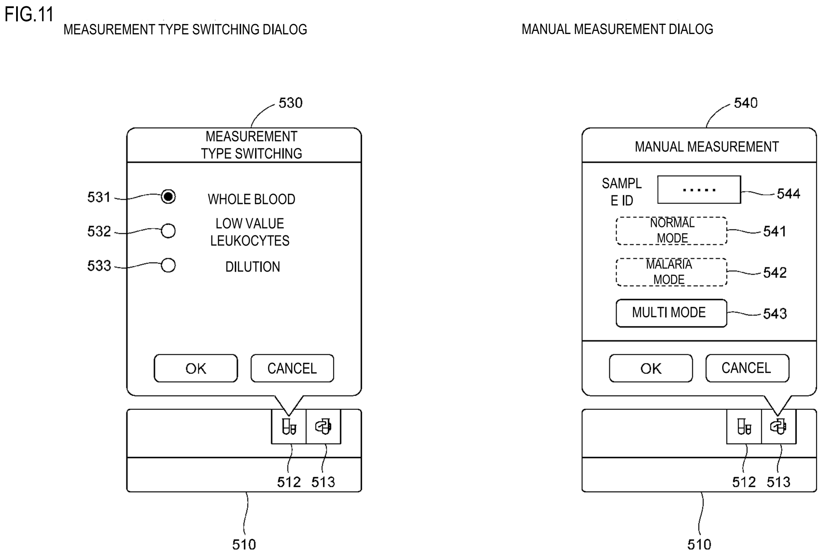

When the controller 401 accepts an instruction to display the menu screen 500, it displays the menu screen 500 on the display 31 and performs processing according to an operation of the operator on the menu screen 500. In the screens shown in FIGS. 11 to 15 below, the controller 401 also displays the screen on the display 31 and performs processing according to an operation of the operator corresponding to the screen.

The menu screen 500 includes a measurement registration button 501, a measurement mode selection button 502, and a measurement unit information display area 510.

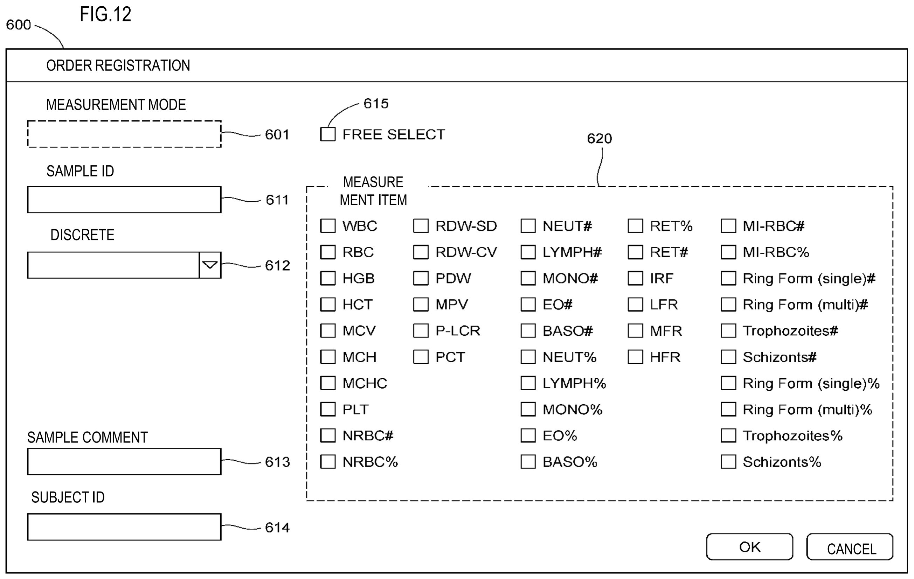

When the measurement registration button 501 is operated, an order registration screen 600, which will be described later, is displayed on the display 31. The order registration screen 600 will be described later with reference to FIGS. 12 to 15.

When the measurement mode selection button 502 is operated, a measurement mode selection dialog 520 is displayed as shown in FIG. 10. The measurement mode selection dialog 520 includes a normal mode button 521, a malaria mode button 522, and a multi mode button 523. When any one of the normal mode button 521, the malaria mode button 522, and the multi mode button 523 is operated, only the operated button is set to a selected state. In FIG. 10, a state where the normal mode button 521 is selected is indicated by a solid line frame, and a state where the malaria mode button 522 and the multi mode button 523 are not selected is indicated by a dashed line frame.

When the OK button is operated after any one of the normal mode button 521, the malaria mode button 522, and the multi mode button 523 is set to a selected state, the controller 401 accepts the selected measurement mode. When the controller 401 accepts the measurement mode, it displays the order registration screen 600 shown in FIG. 12 on the display 31. The acceptance of the measurement mode via the measurement mode selection dialog 520 may be performed for each sample, or the measurement mode once accepted may be applied to a plurality of samples until another measurement mode is accepted.

In the measurement unit information display area 510, various information on the measurement unit 10 (name of the measurement unit 10, sample information during measurement, reagent status, and the like) is displayed. In the measurement unit information display area 510, a sampler measurement button 511 is displayed when measurement of a sample using the conveyer 20 (sampler measurement) is being performed.

As described with reference to FIG. 1, in the sample measurement apparatus 1, in addition to the sampler measurement, a measurement (manual measurement) in which the sample container T is individually supplied to the measurement unit 10 from the front surface of the housing 11 of the measurement unit 10 can also be performed. When the changeover switch 13 of the measurement unit 10 is operated, a cover 15 opens and a sample setting unit 12 is moved forward, and the operator sets the sample container T to be measured in the sample setting unit 12. At this time, as shown in the left part of FIG. 11, a measurement type changeover dialog 530 is displayed above the measurement unit information display area 510.

The left side of FIG. 11 is a diagram showing a configuration of the measurement unit information display area 510 and the measurement type changeover dialog 530 displayed during manual measurement.

During manual measurement, a measurement type changeover button 512 and a manual measurement button 513 are displayed in the measurement unit information display area 510 instead of the sampler measurement button 511 of FIG. 10.

The measurement type changeover dialog 530 includes radio buttons 531 to 533 for determining which of three measurement types (whole blood measurement, low value leukocyte measurement using whole blood, and dilution measurement) a sample to be subjected to manual measurement is to be set. When the OK button is operated, the controller 401 accepts the measurement type of the sample to be subjected to manual measurement according to a selected state of the radio buttons 531 to 533. Then, instead of the measurement type changeover dialog 530, a manual measurement dialog 540 is displayed above the measurement unit information display area 510, as shown in the right part of FIG. 11.

The right side of FIG. 11 is a diagram showing a configuration of the measurement unit information display area 510 and the manual measurement dialog 540 displayed during manual measurement.

The manual measurement dialog 540 includes a normal mode button 541, a malaria mode button 542, and a multi mode button 543, similarly to the measurement mode selection dialog 520 shown in FIG. 10. The manual measurement dialog 540 also includes a sample ID input area 544 which is a text box where a sample ID can be input. When the OK button is operated after a measurement mode is selected and a sample ID is input, the controller 401 accepts the measurement mode and the sample ID, and displays the order registration screen 600 shown in FIG. 12 on the display 31.

Note that the measurement type changeover dialog 530 shown in the left part of FIG. 11 may include a normal mode button, a malaria mode button, and a multi mode button, similarly to the measurement mode selection dialog 520 shown in FIG. 10. In this case, when the OK button is operated after the measurement type and the measurement mode are selected in the measurement type changeover dialog 530, for example, the order registration screen 600 shown in FIG. 12 is displayed without the manual measurement dialog 540 being displayed.

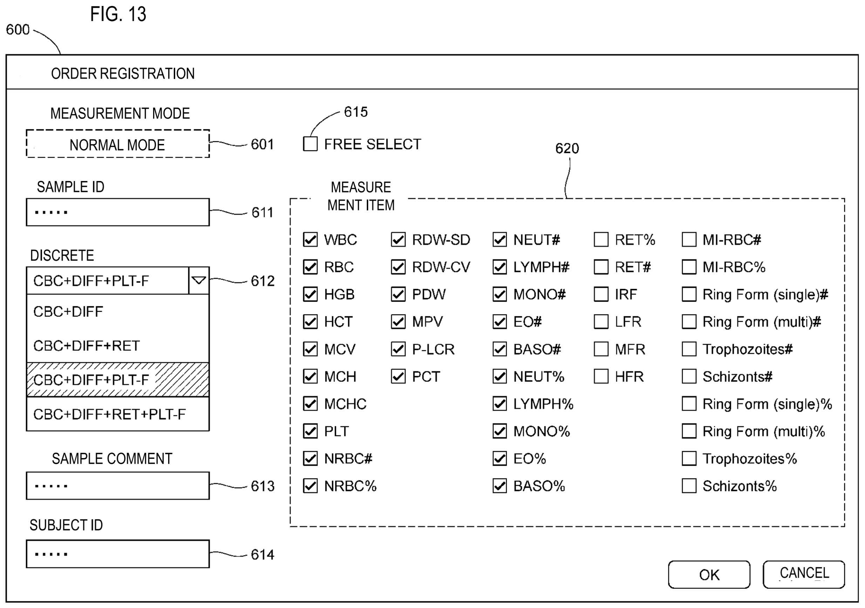

FIG. 12 is a diagram showing a configuration of an order registration screen 600. FIGS. 13 to 15 are diagrams illustrating an example of the order registration screen 600 in a state where the normal mode, the malaria mode, and the multi mode are selected as measurement modes, respectively, and a pull-down menu of a discrete item selection area 612 is displayed.

As shown in FIG. 12, the order registration screen 600 includes a measurement mode display area 601, a sample ID input area 611, a discrete selection area 612, a sample comment input area 613, a subject ID input area 614, a free selection checkbox 615, and a measurement item display area 620.

When the order registration screen 600 is displayed after a measurement mode is set in the measurement mode selection dialog 520 of FIG. 10 or the manual measurement dialog 540 in the right part of FIG. 11, the set measurement mode is automatically displayed in the measurement mode display area 601. Accordingly, as illustrated in FIGS. 13 to 15, the set measurement mode is displayed in the measurement mode display area 601. When a measurement mode is not set, the measurement mode display area 601 is blank.

The order registration screen 600 may be configured such that the measurement mode display area 601 is configured with a pull-down menu so that any of the normal mode, the malaria mode, and the multi mode can be selected.

The sample ID input area 611 is a text box where a sample ID can be input. When a sample ID is input in the manual measurement dialog 540 in the right part of FIG. 11 and the order registration screen 600 is displayed, the input sample ID is automatically displayed in the sample ID input area 611.

The discrete selection area 612 is a pull-down menu from which any of the plurality of discretes shown in FIG. 9 can be selected. Discretes displayed as selection candidates in the discrete item selection area 612 change according to the measurement mode displayed in the measurement mode display area 601.

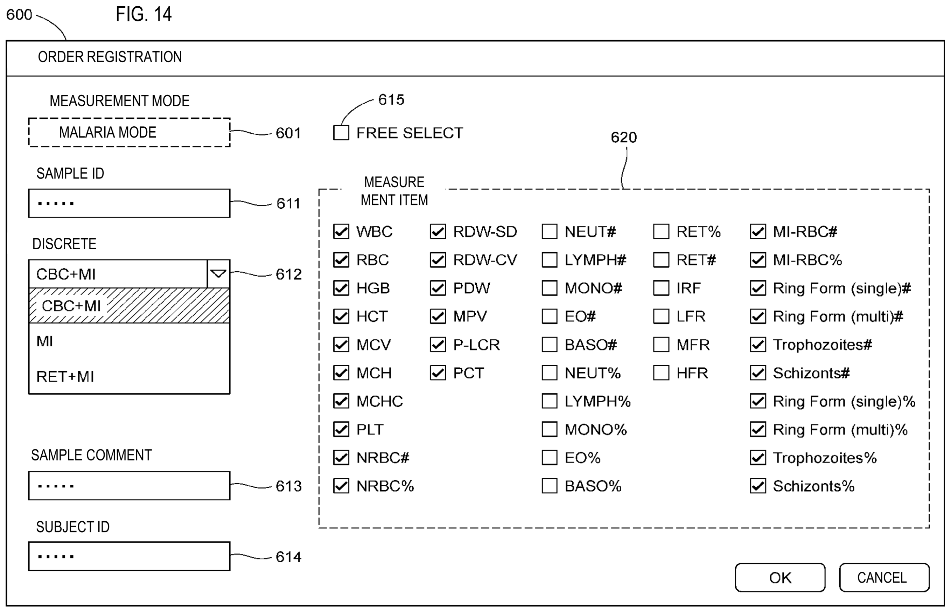

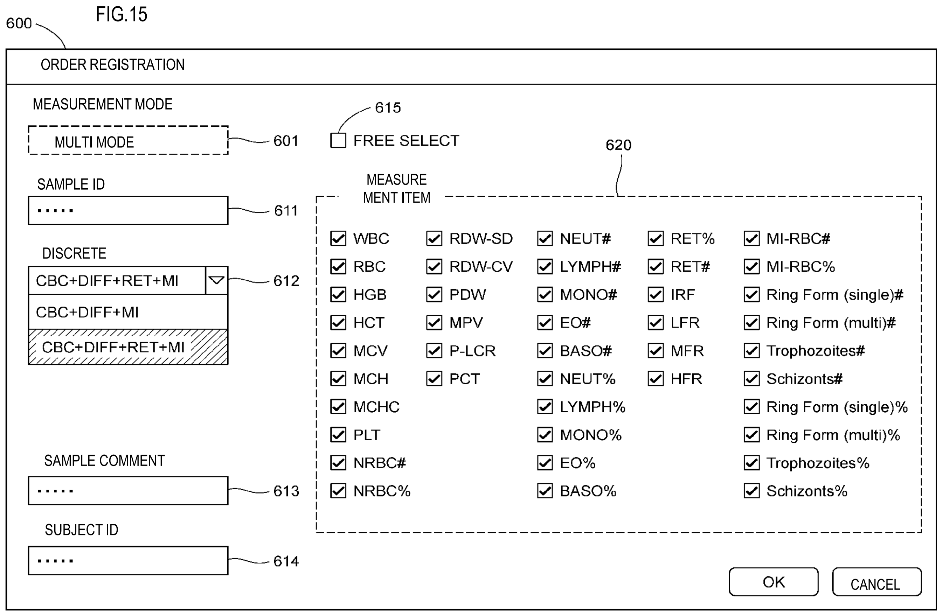

For example, when the measurement mode is the normal mode, four discretes CBC+DIFF, CBC+DIFF+RET, CBC+DIFF+PLT-F, and CBC+DIFF+RET+PLT-F corresponding to the normal mode in FIG. 9 are listed as selection candidates in the discrete selection area 612 as shown in FIG. 13. When the measurement mode is the malaria mode, three discretes CBC+MI, MI, and RET+MI corresponding to the malaria mode in FIG. 9 are listed as selection candidates in the discrete selection area 612 as shown in FIG. 14. When the measurement mode is the multi mode, two discretes CBC+DIFF+MI and CBC+DIFF+RET+MI corresponding to the multi mode in FIG. 9 are listed as selection candidates in the discrete selection area 612 as shown in FIG. 15. When a measurement mode is not selected, all of the thirteen types of discretes shown in FIG. 9 are listed as selection candidates in the discrete selection area 612.

The sample comment input area 613 is a text box where a comment on a sample can be input. In the sample comment input area 613, for example, a medical history of a subject from whom the sample was collected, a travel history to an endemic area of malaria infection, and the like are input. The subject ID input area 614 is a text box where a subject ID can be input.

The measurement item display area 620 shows a selection state of measurement items that can be set in the sample measurement apparatus 1. Each measurement item is provided with a check box, and the check box of a corresponding measurement item is automatically selected according to the discrete selected in the discrete selection area 612. The measurement items selected according to the discrete are predetermined measurement items as described with reference to FIG. 9.

For example, when "CBC+DIFF+PLT-F" is selected in the discrete selection area 612, measurement items are selected in the measurement item display area 620 as shown in FIG. 13. When "CBC+MI" is selected in the discrete selection area 612, measurement items are selected in the measurement item display area 620 as shown in FIG. 14. When "CBC+DIFF+RET+MI" is selected in the discrete selection area 612, measurement items are selected in the measurement item display area 620 as shown in FIG. 15.

The measurement items selected (checked) in the measurement item display area 620 correspond to the discrete selected in the discrete selection area 612 in a one-to-one manner, and cannot be changed by default. On the other hand, when the operator operates the operation unit 32 to check the free selection checkbox 615, the controller 401 may accept the deselection of the measurement item automatically selected according to the selected discrete and the additional selection of unselected measurement items that are not automatically selected according to the selected discrete, by the operation of the operation unit 32. Further, when the free selection checkbox 615 is checked, the controller 401 may accept the selection of a measurement item in the measurement item display area 620 (see FIG. 12) before a discrete is selected, by the operation of the operation unit 32.

When the OK button is operated after a measurement order is input on the order registration screen 600, the controller 401 stores the contents of the order registration screen 600 in the storage 402 as one measurement order. When the sample container T is supplied to the measurement unit 10, the controller 401 performs measurement with the measurement unit 10 and obtains a measurement result based on a measurement order corresponding to the sample in the sample container T, and generates an analysis result based on the measurement result.

Next, a scattergram generated based on the measurement results obtained using the optical detector 110 and analysis of blood cells using the scattergram will be described with reference to FIGS. 16 to 18.

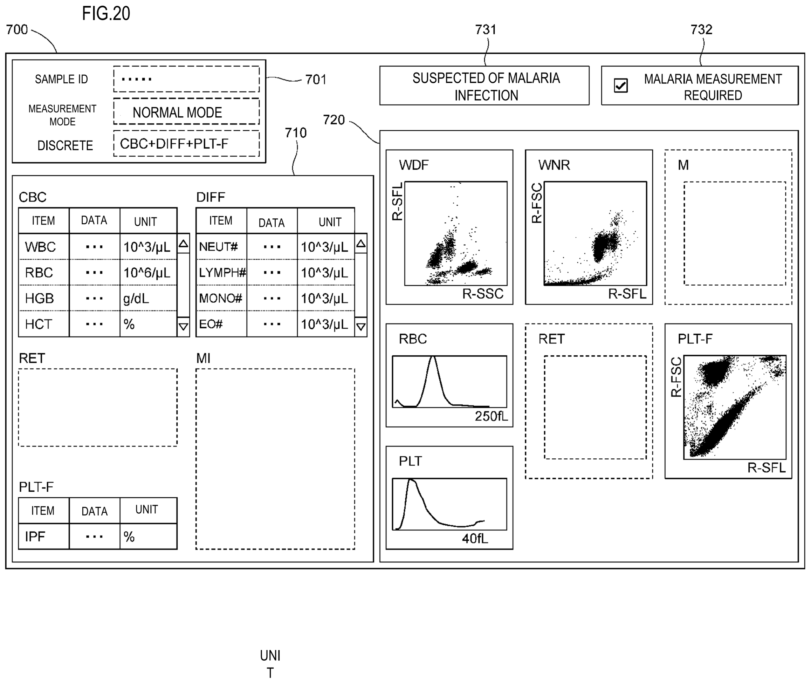

Note that although the scattergrams shown in FIGS. 16 to 18 illustrate plots corresponding to blood cells, these plots are merely for convenience and illustrate a distribution of plots based on a sample. In addition, plots and frequencies of a histogram of scattergrams shown in FIGS. 20 to 22, which will be described later, are also for convenience and illustrate a distribution of plots and frequencies based on a sample.

In the upper part of FIG. 16, a scattergram WDF generated based on the measurement sample WDF is shown. In the scattergram WDF, the horizontal axis corresponds to a level (R-SSC) of an optical signal detected based on side-scattered light generated by light of a wavelength λ20 (red wavelength band) emitted from a light source 202, and the vertical axis corresponds to a level (R-SFL) of an optical signal detected based on fluorescence generated by the light of the wavelength λ20 (red wavelength band) emitted from the light source 202.

The controller 401 plots blood cells using the measurement results based on the measurement sample WDF on the scattergram having the vertical axis and the horizontal axis as described above to generate a scattergram WDF. Then, the controller 401 sets four regions in the scattergram WDF as shown by broken lines, in which (B11) neutrophils and basophils, (B12) lymphocytes, (B13) monocytes, and (B14) eosinophils are distributed, respectively.

In order to set the four regions, the controller 401 performs a classification step of classifying plots corresponding to blood cells as follows. The controller 401 sets initial regions corresponding to blood cells to be classified (in the case of the scattergram WDF, four initial regions for setting (B11) to (B14)). For each of these initial regions, the controller 401 sets plots in the initial region as a cluster belonging to the initial region, and calculates a centroid position of the plots belonging to the cluster. The controller 401 calculates distances from the plots not belonging to the clusters to the centroid positions of the respective clusters, and changes the initial regions such that the plots not belonging to the clusters are included in the cluster with the shortest distance. Then, the controller 401 calculates the centroid positions of the plots belonging to each cluster again, and repeats the above-described processing until a difference between the previous centroid position and the current centroid position is equal to or less than a reference value. Such a classification step is described in, for example, US Patent No. 5555198. US Patent No. 5555198 is incorporated herein by reference.

The controller 401 performs the classification step to classify the blood cells plotted on the scattergram WDF into any of (B11) to (B14) and obtains the number of blood cells for each region. As a result, leukocytes are classified, and the total number of neutrophils and basophils, the number of lymphocytes, the number of monocytes, and the number of eosinophils are obtained.

In the lower part of FIG. 16, a scattergram WNR generated based on the measurement sample WNR is shown. In the scattergram WNR, the horizontal axis corresponds to a level (R-SFL) of an optical signal detected based on fluorescence generated by light of a wavelength λ20 (red wavelength band) emitted from the light source 202, and the vertical axis corresponds to a level (R-FSC) of an optical signal detected based on forward-scattered light generated by the light of the wavelength λ20 (red wavelength band) emitted from the light source 202.

The controller 401 plots blood cells using the measurement results based on the measurement sample WNR on the scattergram having the vertical axis and the horizontal axis as described above to generate a scattergram WNR. Then, the controller 401 performs the classification step to set three regions in the scattergram WNR as shown by broken lines, in which (B21) nucleated red blood cells, (B22) basophils, and (B23) neutrophils, lymphocytes, monocytes, and eosinophils are distributed, respectively. The controller 401 obtains the number of blood cells for each of the regions (B21) to (B23). In addition, the controller 401 calculates the total number of blood cells of (B22) and (B23) to obtain the number of leukocytes. As a result, the total number of neutrophils, lymphocytes, monocytes, and eosinophils, and the numbers of nucleated red blood cells, basophils, and leukocytes are obtained.

The controller 401 obtains a difference between the number of blood cells of (B11) and the number of blood cells of (B22) as the number of neutrophils. As a result, the controller 401 counts leukocytes in a target sample and classifies the leukocytes into five, which are neutrophils, lymphocytes, monocytes, eosinophils, and basophils, to obtain the number of blood cells for each of the five classifications. Note that when the classification of neutrophils and basophils is unnecessary, the preparation of the measurement sample WNR and the creation of the scattergram WNR may be omitted. In this case, the controller 401 may obtain the total number of blood cells of (B11) to (B14) as the number of leukocytes.

In the upper part of FIG. 17, a scattergram M generated based on the measurement sample M is shown. In the scattergram M, the horizontal axis corresponds to a level (V-SFL) of an optical signal detected based on fluorescence generated by light of a wavelength λ10 (blue-violet wavelength band) emitted from the light source 201, and the vertical axis corresponds to a level (R-FSC) of an optical signal detected based on forward-scattered light generated by light of a wavelength λ20 (red wavelength band) emitted from the light source 202. The vertical axis may correspond to a level (V-FSC) of an optical signal detected based on forward-scattered light generated by the light of the wavelength λ10 (blue-violet wavelength band) emitted from the light source 201.

The controller 401 plots blood cells using the measurement results based on the measurement sample M on the scattergram having the vertical axis and the horizontal axis as described above to generate a scattergram M. Then, the controller 401 performs the classification step to set three regions in the scattergram M as shown by broken lines, in which (B30) red blood cells infected with malaria, (B31) red blood cells not infected with malaria, and (B36) leukocytes are distributed, respectively. The controller 401 may set four regions as subpopulations of red blood cells infected with malaria, in which (B32) ring forms (single), (B33) ring forms (multi), (B34) trophozoites, and (B35) schizonts are distributed in the region of (B30), respectively. The controller 401 obtains the number of blood cells for each of the regions (B30), (B31), and (B36), or the number of blood cells for each of the regions (B31) to (B36). As a result, the number of red blood cells infected with malaria, the number of red blood cells not infected with malaria, and the number of leukocytes, or the number of red blood cells infected with malaria, the number of ring forms (single), the number of ring forms (multi), the number of trophozoites, the number of schizonts, the number of red blood cells not infected with malaria, and the number of leukocytes are obtained.

A ring form is a red blood cell infected with a malaria parasite in the form of a ring form. A ring form (single) is a red blood cell containing one malaria parasite in a ring form. A ring form (multi) is a red blood cell containing a plurality of malaria parasites in a ring form. A trophozoite is a red blood cell infected with a malaria parasite in the form of a trophozoite. A schizont is a red blood cell infected with a malaria parasite in the form of a schizont.

When a merozoite, which is one of the forms of malaria parasites, invades a red blood cell, an intraerythrocytic life cycle of the malaria parasite begins. In the intraerythrocytic life cycle, the form of the malaria parasite changes in the order of ring form, trophozoite, and schizont. Schizonts divide into a plurality of merozoites and destroy red blood cells at that time. As a result, a large number of merozoites are released into the blood. The merozoites invade the next red blood cell, and the intraerythrocytic life cycle starts again. The malaria parasites multiply by repeating such a cycle.

As described above, the controller 401 analyzes cells (blood cells) by clustering using scattergrams. The horizontal axis, the vertical axis, and the initial region in the scattergram WDF, the scattergram WNR, and the scattergram M are different from each other. That is, the controller 401 applies different clustering to the scattergram WDF, the scattergram WNR, and the scattergram M to analyze cells.

In the lower part of FIG. 17, a scattergram RET generated based on the measurement sample RET is shown. In the scattergram RET, the horizontal axis corresponds to a level (R-SFL) of an optical signal detected based on fluorescence generated by light of a wavelength λ20 (red wavelength band) emitted from the light source 202, and the vertical axis corresponds to a level (R-FSC) of an optical signal detected based on forward-scattered light generated by the light of the wavelength λ20 (red wavelength band) emitted from the light source 202.

The controller 401 plots blood cells using the measurement results based on the measurement sample RET on the scattergram having the vertical axis and the horizontal axis as described above to generate a scattergram RET. Then, the controller 401 performs the classification step to set three regions in the scattergram RET as shown by broken lines, in which (B41) mature red blood cells, (B42) reticulocytes, and (B46) platelets are distributed, respectively. The controller 401 may divide the (B42) reticulocytes based on a size of R-SFL, and set three regions in which (B43) low fluorescent reticulocytes, (B44) medium fluorescent reticulocytes, and (B45) high fluorescent reticulocytes are distributed, respectively. The controller 401 obtains the number of blood cells for each of the regions (B41), (B42), and (B46), or the number of blood cells for each of the regions (B41) to (B46). As a result, the number of mature red blood cells, the number of reticulocytes, and the number of platelets, or the number of mature red blood cells, the number of reticulocytes, the number of low fluorescent reticulocytes, the number of medium fluorescent reticulocytes, the number of high fluorescent reticulocytes, and the number of platelets are obtained.

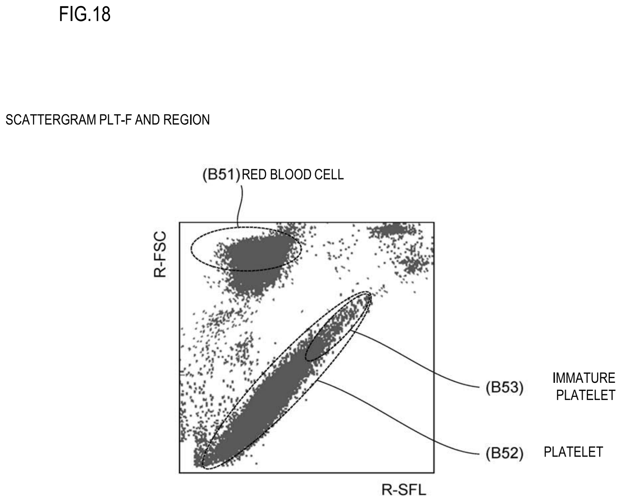

FIG. 18 shows a scattergram PLT-F generated based on the measurement sample PLT-F. In the scattergram PLT-F, the horizontal axis corresponds to a level (R-SFL) of an optical signal detected based on fluorescence generated by light of a wavelength λ20 (red wavelength band) emitted from the light source 202, and the vertical axis corresponds to a level (R-FSC) of an optical signal detected based on forward-scattered light generated by the light of the wavelength λ20 (red wavelength band) emitted from the light source 202.

The controller 401 plots blood cells using the measurement results based on the measurement sample PLT-F on the scattergram having the vertical axis and the horizontal axis as described above to generate a scattergram PLT-F. Then, the controller 401 performs the classification step to set two regions in the scattergram PLT-F as shown by broken lines, in which (B51) red blood cells and (B52) platelets are distributed, respectively. The controller 401 also sets a region in which (B53) immature platelets are distributed in (B52). The controller 401 obtains the number of blood cells for each of the regions (B51) to (B53). As a result, the number of red blood cells, the number of platelets, and the number of immature platelets are obtained.

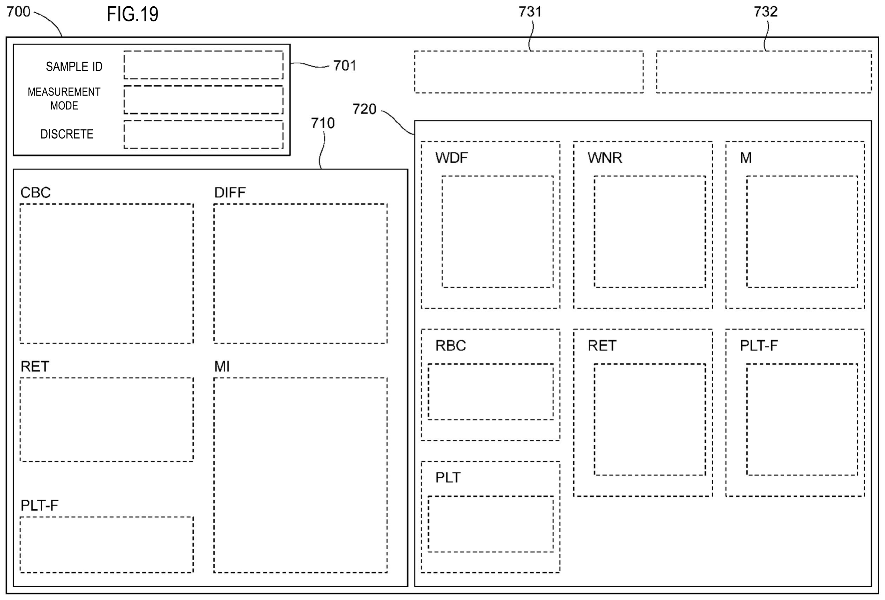

FIG. 19 is a diagram showing a configuration of an analysis result display screen 700. FIGS. 20 to 22 are diagrams illustrating the analysis result display screen 700 when the measurement mode and the discrete are set as shown in FIGS. 13 to 15, respectively.

When the controller 401 accepts an instruction to display the analysis result display screen 700, it displays the analysis result display screen 700 on the display 31 and performs processing according to an operation of the operator on the analysis result display screen 700. The display content of the analysis result display screen 700 is based on an analysis result corresponding to one sample ID.

As shown in FIG. 19, the analysis result display screen 700 includes an analysis information display area 701, a result value display area 710, a graph display area 720, a flag display area 731, and a measurement necessity setting area 732.

In the analysis information display area 701, a sample ID, a measurement mode, and a discrete are displayed. In the result value display area 710, lists corresponding to the sub-discretes CBC, DIFF, MI, RET, and PLT-F, respectively, are displayed. In each list, a result value of a measurement item is displayed. In the graph display area 720, a scattergram WDF, a scattergram WNR, a scattergram M, a scattergram RET, a scattergram PLT-F, a histogram RBC, and a histogram PLT are displayed. The histogram RBC is a histogram regarding red blood cells generated based on the measurement sample RBC/PLT, and the histogram PLT is a histogram regarding platelets generated based on the measurement sample RBC/PLT.

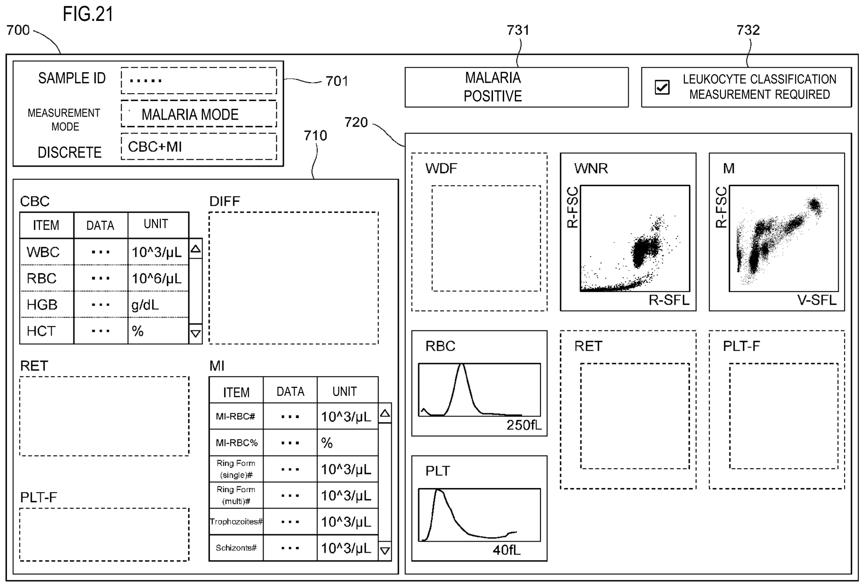

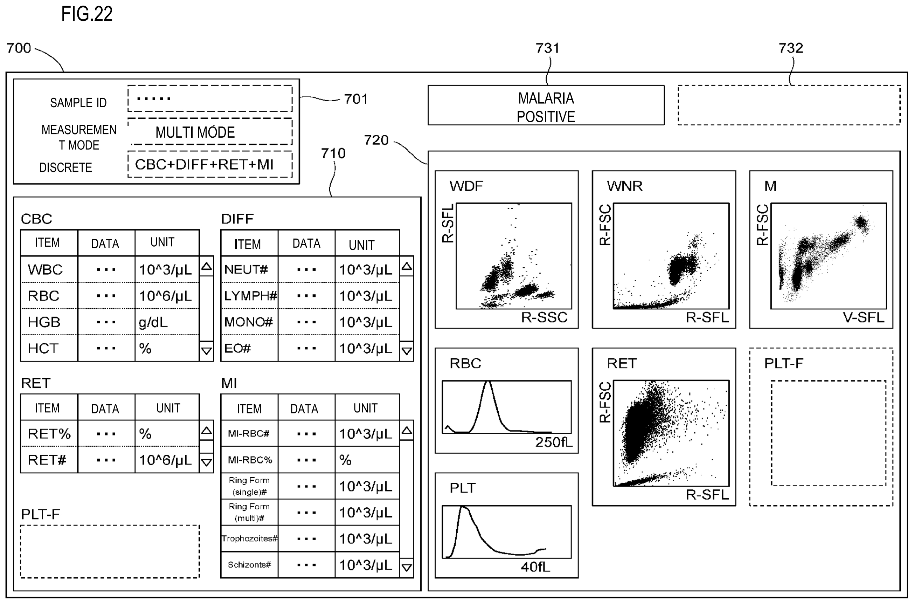

For example, when the discrete is "CBC+DIFF+PLT-F," as shown in FIG. 20, a list showing result values of CBC, DIFF, and PLT-F is displayed in the result value display area 710, and a scattergram WDF, a scattergram WNR, a scattergram PLT-F, a histogram RBC, and a histogram PLT are displayed in the graph display area 720. When the discrete is "CBC+MI," as shown in FIG. 21, a list showing result values of CBC and MI is displayed in the result value display area 710, and a scattergram WNR, a scattergram M, a histogram RBC, and a histogram PLT are displayed in the graph display area 720. When the discrete is "CBC+DIFF+RET+MI," as shown in FIG. 22, a list showing result values of CBC, DIFF, RET, and MI is displayed in the result value display area 710, and a scattergram WDF, a scattergram WNR, a scattergram M, a scattergram RET, a histogram RBC, and a histogram PLT are displayed in the graph display area 720.

In the flag display area 731, when a predetermined disease is suspected based on the analysis results, a message indicating that the predetermined disease is suspected is displayed.

For example, when the measurement mode is the normal mode, if the controller 401 determines that a plot group exists in a region to the left of the region (B11) and below the region (B12) in the scattergram WDF shown in the upper part of FIG. 16, it adds a flag of red blood cells infected with malaria to the analysis result. In this case, as shown in FIG. 20, "malaria infection suspected" is displayed in the flag display area 731. For example, when the measurement mode is the malaria mode or the multi mode, if the controller 401 determines that the number of red blood cells infected with malaria (MI-RBC#) in the result value display area 710 is equal to or greater than a predetermined value, it adds a flag of malaria positive to the analysis result. In this case, as shown in FIGS. 21 and 22, "malaria positive" is displayed in the flag display area 731.

When a predetermined disease is suspected in the flag display area 731, the measurement necessity setting area 732 displays a name of a measurement for performing a detailed examination of the predetermined disease and a checkbox for setting whether or not to perform the measurement.

For example, when the measurement mode is a normal mode, when the controller 401 causes the flag display area 731 to display "suspected of malaria infection," as shown in FIG. 20, the controller 401 causes the measurement necessity setting area 732 to display a "malaria measurement required" message and a check box. In this case, if the operator operates the check box and sets it to a checked state, a flag indicating that it is preferable to perform a measurement related to malaria (for example, discrete MI measurement) is added to the analysis result. When the measurement mode is a malaria mode, when the controller 401 causes the flag display area 731 to display "malaria positive," as shown in FIG. 21, the controller 401 causes the measurement necessity setting area 732 to display a "leukocyte classification measurement required" message and a check box. In this case, if the operator operates the check box and sets it to a checked state, a flag indicating that it is preferable to perform a measurement related to leukocyte classification (for example, discrete CBC+DIFF measurement) is added to the analysis result.

When the measurement necessity setting area 732 is set to a checked state for a certain sample and a flag indicating that it is preferable to perform measurement regarding malaria or measurement regarding leukocyte classification is added to the analysis results, a doctor or another operator can know that a necessary measurement is needed for the sample when referring to the analysis result display screen 700 for the sample. As a result, the doctor or other operator can smoothly perform the necessary measurement.

When the measurement mode is the multi-mode, the analysis result display screen 700 shown in FIG. 22 may be divided into a screen regarding classification of leukocytes and a screen regarding malaria-infected red blood cells. In this case, for example, a result value other than the discrete MI and a scattergram are displayed on the screen regarding classification of leukocytes, and only the result value of the discrete MI and a scattergram are displayed on the screen regarding malaria-infected red blood cells. The histogram RBC and the histogram PLT may be displayed on at least one of the screens. Also in this case, a button for switching and displaying the respective screens may be arranged on both the screen regarding classification of leukocytes and the screen regarding malaria-infected red blood cells.

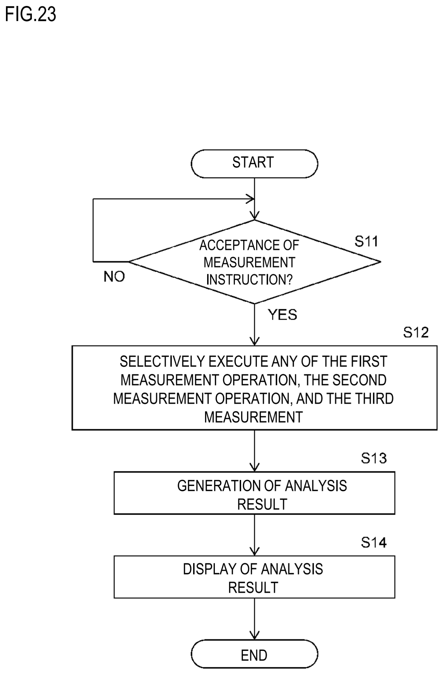

FIG. 23 is a flowchart showing a process performed by a controller 401 of an analysis unit 30.

In step S11, the controller 401 stands by until it accepts a measurement instruction. In the case of a sampler mode, when a sample rack R is set in a conveyer 20, the controller 401 accepts a measurement instruction by receiving information indicating that the sample rack R has been set from the conveyer 20. In the case of a manual mode, when a start switch 14 is operated, the controller 401 accepts a measurement instruction by receiving information indicating that the start switch 14 has been operated from the conveyer 20. When the measurement instruction is accepted (Step S11: YES), the controller 401 transfers a sample container T into a housing 11 and controls a reader 161 to read a sample ID from the sample container T. The controller 401 reads out a measurement order stored in a storage 402 based on the read sample ID.

In the case of the sampler mode, the controller 401 repeatedly performs the processes of the following steps S12 and S13 for each of a plurality of samples continuously supplied to the measurement unit 10, and in the case of the manual mode, performs the processes of the following steps S12 and S13 for the sample set in the sample setting unit 12.

In step S12, the controller 401 controls the measurement unit 10 to selectively execute any one of a first measurement operation, a second measurement operation, and a third measurement operation based on a measurement order. The first measurement operation is a measurement operation for leukocyte classification, which is performed when the normal mode discrete shown in FIG. 9 is selected. The second measurement operation is a measurement operation for counting malaria-infected red blood cells, which is performed when the malaria mode discrete shown in FIG. 9 is selected. The third measurement operation is a measurement operation for leukocyte classification and counting of malaria-infected red blood cells, which is performed when the multi-mode discrete shown in FIG. 9 is selected. In step S12, the controller 401 may read out a measurement order input via the order registration screen 600 and stored in the storage 402, or may receive a measurement order transmitted from a host computer capable of communicating with the analysis unit 30.

That is, the controller 401 controls the measurement unit 10 to selectively execute a plurality of measurement operations including: a first measurement operation for measuring a sample using a staining reagent WDF containing a fluorescent dye that stains leukocytes for leukocyte classification; a second measurement operation for measuring a sample using a staining reagent M containing a fluorescent dye that stains red blood cells suspected of malaria infection; and a third measurement operation for measuring a sample using the staining reagent WDF and the staining reagent M.

In the discrete CBC, CBC+RET, CBC+PLT-F, and CBC+RET+PLT-F of FIG. 9, the measurement unit 10 executes only other measurement operations that do not correspond to any of the first measurement operation, the second measurement operation, and the third measurement operation. That is, in the present embodiment, the measurement unit 10 can selectively execute a plurality of measurement operations including the first measurement operation, the second measurement operation, the third measurement operation, and other measurement operations on the sample.

The measurement unit 10 generates a measurement result by the measurement operation in step S12 and transmits the generated measurement result to the analysis unit 30. In step S13, the controller 401 performs an analysis based on the measurement result received from the measurement unit 10 and generates an analysis result.

After that, when the controller 401 accepts a display instruction of the analysis result display screen 700 from an operator, in step S14, the controller 401 displays the analysis result display screen 700 on the display 31 based on the analysis result of the target sample ID.

### "<Effects of Sample Measurement Apparatus According to First Embodiment>

A sample measurement apparatus 1 configured to measure a sample collected from a subject includes: a measurement unit 10 configured to prepare a measurement sample WDF, a measurement sample M, and the like from the sample and a reagent, and to detect at least optical signals corresponding to cells in the measurement sample; and an analysis unit 30 configured to analyze the cells according to measurement of the sample by the measurement unit 10. The measurement unit 10 is selectively executable with a plurality of measurement operations including: (1) a first measurement operation for measuring the sample using a staining reagent WDF (first reagent) containing a fluorescent dye (first fluorescent dye) that stains leukocytes to classify the leukocytes; (2) a second measurement operation for measuring the sample using a staining reagent M (second reagent) containing a fluorescent dye (second fluorescent dye) that stains cells suspected of malaria infection; and (3) a third measurement operation for measuring the sample using the staining reagent WDF (first reagent) and the staining reagent M (second reagent)."

According to this configuration, for example, either one or both of the measurement for leukocyte classification and the measurement for malaria-infected red blood cells can be appropriately selected and executed according to a measurement order, an analysis result, a medical history, a test result of another apparatus, a history of travel to a malaria-endemic area, a period such as the rainy season when malaria is likely to be prevalent, and the like. As a result, sample inspection operations including malaria infection tests can be made more efficient.

The measurement unit 10 includes an optical detector 110, an electrical detector 120, a hemoglobin detector 130, a reader 161, a sample container transfer unit 162, a dispensing unit 163, liquid transfer units 164 and 165, light sources 201 and 202, an aspiration tube 301, syringe pumps 311 and 342, and chambers C11 to C14, C21, and C22 (plurality of mechanisms configured to measure a measurement sample). The measurement unit 10 shares at least one of the plurality of mechanisms among the first measurement operation, the second measurement operation, and the third measurement operation.

According to this configuration, the configuration of the sample measurement apparatus 1 can be simplified as compared to a case where a mechanism is provided for each measurement operation.

The measurement unit 10 includes an optical detector 110 configured to detect optical signals corresponding to cells in a measurement sample. The measurement unit 10 shares the optical detector 110 among the first measurement operation, the second measurement operation, and the third measurement operation.

According to this configuration, the configuration of the sample measurement apparatus 1 can be simplified as compared to a case where an optical detector is provided for each measurement operation.

The measurement unit 10 includes light sources 201 and 202 (at least one light source) configured to irradiate a measurement sample with light. The measurement unit 10 shares the at least one light source among the first measurement operation, the second measurement operation, and the third measurement operation.

According to this configuration, the configuration of the sample measurement apparatus 1 can be simplified as compared to a case where a light source is provided for each measurement operation.

The measurement unit 10 includes a light source 201 configured to irradiate a measurement sample with light of a wavelength λ10 (first wavelength) and a light source 202 configured to irradiate the measurement sample with light of a wavelength λ20 (second wavelength). In the first measurement operation, the measurement unit 10 irradiates the measurement sample with at least one of the light of the wavelength λ10 (first wavelength) and the light of the wavelength λ20 (second wavelength), and in the second measurement operation, irradiates the measurement sample with the light of the wavelength λ10 (first wavelength) and the light of the wavelength λ20 (second wavelength).

According to this configuration, the configuration of the sample measurement apparatus 1 can be simplified as compared to a case where a light source is provided corresponding to each of the first measurement operation and the second measurement operation.

The measurement unit 10 includes an aspiration tube 301 configured to aspirate a sample supplied to the sample measurement apparatus 1. The measurement unit 10 shares the aspiration tube 301 among the first measurement operation, the second measurement operation, and the third measurement operation.

According to this configuration, the configuration of the sample measurement apparatus 1 can be simplified as compared to a case where an aspiration tube is provided for each measurement operation.

The measurement unit 10 includes an aspiration tube 301 configured to aspirate a sample supplied to the sample measurement apparatus 1 and a syringe pump 311 (pump) used to aspirate the sample with the aspiration tube 301. The measurement unit 10 shares the aspiration tube 301 and the syringe pump 311 (pump) among the first measurement operation, the second measurement operation, and the third measurement operation.

According to this configuration, the configuration of the sample measurement apparatus 1 can be simplified as compared to a case where an aspiration tube and a pump are provided for each measurement operation.

The measurement unit 10 executes the first measurement operation and the second measurement operation in the third measurement operation.

According to this configuration, it is possible to enhance the possibility of obtaining a more accurate analysis result as compared to a case where a measurement sample WDF-M1 containing both a staining reagent WDF (first reagent) and a staining reagent M (second reagent) is measured in the third measurement operation, as in a modification example 2 described later.

The measurement unit 10 includes an optical detector 110, an electrical detector 120, a hemoglobin detector 130, a reader 161, a sample container transfer unit 162, a dispensing unit 163, liquid transfer units 164 and 165, light sources 201 and 202, an aspiration tube 301, syringe pumps 311 and 342, and chambers C11 to C14, C21, and C22 (a plurality of mechanisms configured to measure a measurement sample). At least one of the plurality of mechanisms performs different operations in each of the first measurement operation and the second measurement operation.

For example, in the first embodiment, the amount of the sample dispensed in the first measurement operation is different from the amount of the sample dispensed in the second measurement operation, and the measurement time of the optical detector 110 in the first measurement operation is different from the measurement time of the optical detector 110 in the second measurement operation. Therefore, the syringe pumps 311 and 342 perform different operations in each of the first measurement operation and the second measurement operation. According to the above configuration, in the first measurement operation and the second measurement operation, the target mechanism can be caused to perform an appropriate operation corresponding to the measurement operation.

The measurement unit 10 includes an optical detector 110 configured to detect optical signals corresponding to cells in a measurement sample. A measurement time by the optical detector 110 in the first measurement operation is different from a measurement time by the optical detector 110 in the second measurement operation.

According to this configuration, in the first measurement operation and the second measurement operation, the optical detector 110 can be caused to perform measurement for an appropriate measurement time corresponding to the measurement operation.

The measurement unit 10 includes chambers C11 to C14, C21, and C22 configured to prepare a measurement sample from a sample and a reagent, and a syringe pump 311 or the like (a sample preparation unit). The chambers C11 and C13, the syringe pump 311, and the like (a sample preparation unit) prepare a measurement sample WDF (first measurement sample) from the sample and a staining reagent WDF (first reagent) in the first measurement operation, and prepare a measurement sample M (second measurement sample) from the sample and a staining reagent M (second reagent) in the second measurement operation.

According to this configuration, the measurement sample WDF and the measurement sample M can be smoothly prepared in the first measurement operation and the second measurement operation, respectively.

The chambers C11 and C13 (a sample preparation unit) prepare a measurement sample WDF (first measurement sample) from the sample, a staining reagent WDF (first reagent), and a hemolytic reagent WDF (first hemolytic reagent) in the first measurement operation, and prepare a measurement sample M (second measurement sample) from the sample, a staining reagent M (second reagent), and a hemolytic reagent M (second hemolytic reagent) in the second measurement operation.

According to this configuration, in the first measurement operation, the measurement sample WDF can be appropriately prepared using the hemolytic reagent WDF, and in the second measurement operation, the measurement sample M can be appropriately prepared using the hemolytic reagent M.

The measurement unit 10 measures a sample in the second measurement operation for counting cells suspected of malaria infection (counting blood cells in an area (B30) of a scattergram M) and counting leukocytes (counting in an area (B36) of the scattergram M).

According to this configuration, based on a measurement result obtained by the second measurement operation, a malaria test can be performed with high accuracy by referring to the number of malaria-infected red blood cells and the number of leukocytes.

The measurement unit 10 includes an optical detector 110 configured to detect optical signals corresponding to cells in a measurement sample and an electrical detector 120 configured to detect electrical signals corresponding to the cells in the measurement sample. The measurement unit 10 uses both the optical detector 110 and the electrical detector 120 in the first measurement operation and the second measurement operation.

According to this configuration, in the first measurement operation and the second measurement operation, the sample can be analyzed in more detail by using both the optical detector 110 and the electrical detector 120. Specifically, the optical detector 110 can perform classification and counting of leukocytes, and the electrical detector 120 can perform counting of red blood cells and platelets.

The analysis unit 30 analyzes cells by different clustering in the first measurement operation and the second measurement operation.

According to this configuration, appropriate cell analysis can be performed in each of the first measurement operation and the second measurement operation.

The analysis unit 30 classifies leukocytes into a plurality of subpopulations in analysis corresponding to the first measurement operation.

According to this configuration, a test based on leukocytes can be performed with high accuracy.

The analysis unit 30 obtains information regarding a malaria life cycle for cells suspected of malaria infection in analysis corresponding to the second measurement operation.

According to this configuration, since information regarding the malaria life cycle (life stage) such as ring form, merozoite, and schizont is obtained, the state of malaria parasites infecting the subject can be grasped in detail.

The analysis unit 30 selects a measurement operation to be executed by the measurement unit 10 according to a measurement order (information regarding a sample) (step S12 in FIG. 23).

According to this configuration, since the first, second, and third measurement operations are automatically selected according to a measurement order, the measurement operations can be smoothly executed by registering the measurement order in advance.

The analysis unit 30 includes a controller 401, a display 31, and an operation unit 32. The controller 401 causes the display 31 to display a measurement mode selection dialog 520 and a manual measurement dialog 540 (screens) for selecting one measurement mode from three measurement modes that classify a plurality of measurement operations into the first, second, and third measurement operations, respectively, accepts the measurement mode selected via the measurement mode selection dialog 520 and the manual measurement dialog 540 (screens) by operation of the operation unit 32, and causes the display 31 to display an order registration screen 600 (another screen) for selecting a measurement operation corresponding to the accepted measurement mode.

According to this configuration, an operator can display the order registration screen 600 for selecting a measurement operation corresponding to a measurement mode by selecting the measurement mode. As a result, the operator can smoothly select a measurement operation via the order registration screen 600.

The analysis unit 30 includes a controller 401, a display 31, and an operation unit 32. The controller 401 causes the display 31 to display an order registration screen 600 (screen) for selecting the first, second, and third measurement operations from a plurality of measurement operations, respectively, and accepts a measurement operation selected via the order registration screen 600 (screen) by operation of the operation unit 32.

According to this configuration, an operator can cause the measurement unit 10 to execute a measurement operation necessary for a subject.

The analysis unit 30 includes a controller 401, a display 31, and an operation unit 32. The controller 401 may cause the display 31 to display an order registration screen 600 (screen) for selecting at least one measurement item from a plurality of measurement items that respectively define the first, second, and third measurement operations, and accept the measurement item selected via the order registration screen 600 (screen) by operation of the operation unit 32.

According to this configuration, an operator can set a measurement operation necessary for a subject in more detail and can cause the measurement unit 10 to execute the set measurement operation.

The analysis unit 30 includes a controller 401, a display 31, and an operation unit 32. The controller 401 causes the display 31 to display an order registration screen 600 (screen) for registering a measurement order indicating which of the first, second, and third measurement operations is to be executed, and accepts the measurement order registered via the order registration screen 600 (screen) by operation of the operation unit 32.

According to this configuration, by registering a measurement order in advance, a measurement operation necessary for a subject can be smoothly executed thereafter.

### "<Embodiment 2>

Based on an analysis result obtained by measurement by the sample measurement apparatus 1, it may be determined whether or not the sample measurement apparatus 1 performs measurement on the same sample for another discrete (measurement item), that is, whether or not a reflex measurement is necessary. In this case, when it is determined that a reflex measurement is necessary, a target measurement is automatically performed. Hereinafter, a configuration and a process different from those of the first embodiment will be described."

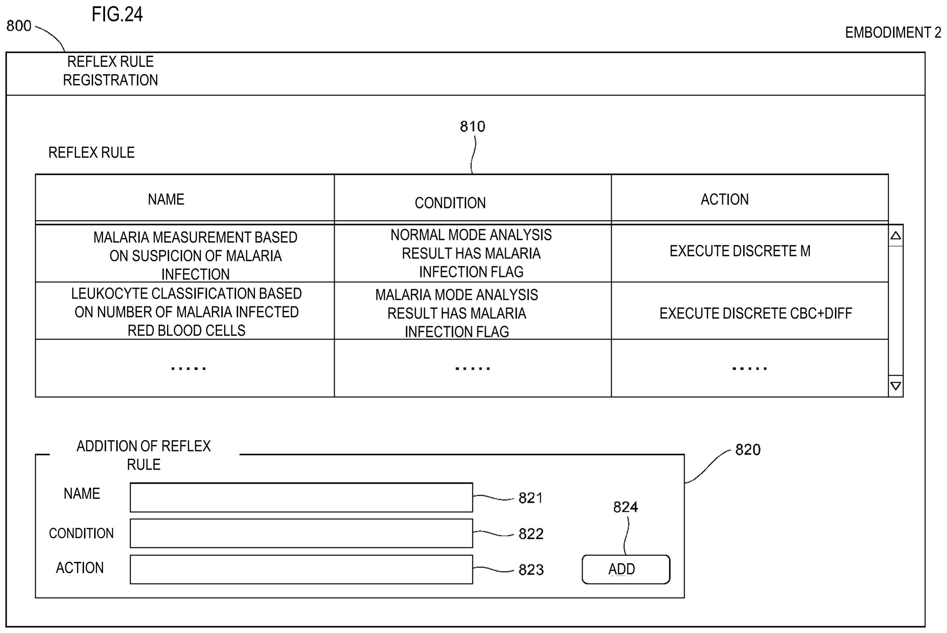

FIG. 24 is a diagram showing a configuration of a reflex setting screen 800 according to the present embodiment.

When the controller 401 accepts a display instruction of the reflex setting screen 800, the controller 401 displays the reflex setting screen 800 on the display 31 and performs a process according to an operation of an operator on the reflex setting screen 800.

The reflex setting screen 800 includes a reflex rule display area 810 and a reflex rule addition area 820.

A reflex rule for determining whether or not a reflex measurement is necessary is displayed on the reflex rule display area 810. Each row corresponds to one reflex rule. A reflex rule includes a name, a conditional expression for determining that a reflex measurement is necessary, and an action defining a process to be automatically performed when it is determined that a reflex measurement is necessary.

A reflex rule includes executing a discrete MI when a flag of malaria-infected red blood cells is added to an analysis result of a normal mode, for example, as shown in a first row of the reflex rule display area 810. In this case, since the number of malaria-infected red blood cells, which could not be obtained by leukocyte classification, can be obtained, it is possible to accurately determine whether or not a subject is suffering from malaria. Further, a reflex rule includes executing a discrete CBC+DIFF when a flag of malaria-positive is added to an analysis result of a malaria mode, for example, as shown in a second row of the reflex rule display area 810. In this case, since the number of leukocytes for each classification, which could not be obtained by classification of malaria-infected red blood cells, can be obtained, the state of the subject can be accurately grasped.

The reflex rule addition area 820 includes text boxes 821 to 823 corresponding to a name, a conditional expression, and an action of a reflex rule, respectively, and an addition button 824. When a name, a conditional expression, and an action are input via the operation unit 32 and the addition button 824 is operated, the controller 401 accepts the contents of the reflex rule addition area 820, stores the contents in the storage 402, and adds and displays the accepted reflex rule in the reflex rule display area 810.

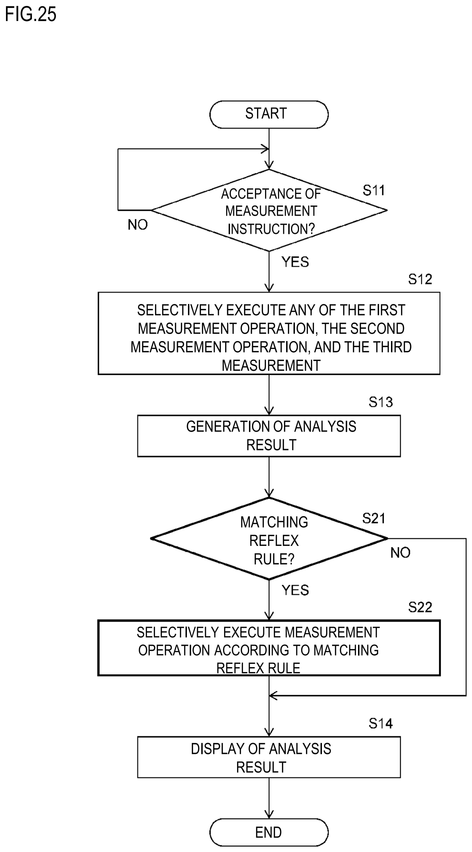

FIG. 25 is a flowchart showing a process performed by the controller 401 of the analysis unit 30.

In the process of FIG. 25, steps S21 and S22 are added as compared to the process of the first embodiment of FIG. 23. In step S21, the controller 401 determines whether or not there is an analysis result that matches a conditional expression of a reflex rule stored in the storage 402 among analysis results generated in step S13. When there is an analysis result that matches the conditional expression of the reflex rule (step S21: YES), in step S22, the controller 401 transfers the sample container T containing the sample that is the determination target of the reflex rule to the sample aspiration position in the housing 11 again. Then, the controller 401 selectively executes a measurement operation on the sample in the sample container T according to an action of the matched reflex rule.

### "<Effects of Sample Measurement Apparatus According to Second Embodiment>

The analysis unit 30 selects a measurement operation to be executed by the measurement unit 10 according to an analysis result (information regarding a sample) (step S22 in FIG. 25)."

According to this configuration, since the first, second, and third measurement operations are automatically selected according to an analysis result, it is possible to save the trouble of an operator selecting a measurement operation by referring to the analysis result.

### "<Modification Example 1>

In the first embodiment, a measurement operation is selectively executed based on a measurement order, and in the second embodiment, a measurement operation is selectively executed again based on an analysis result. However, the measurement operation may be selectively executed based on other information regarding a sample, not limited to information regarding a sample such as a measurement order and an analysis result. For example, other information regarding a sample is information regarding a subject from whom the sample is collected, such as a medical history, a test result of another apparatus, and a history of travel to a malaria-endemic area."

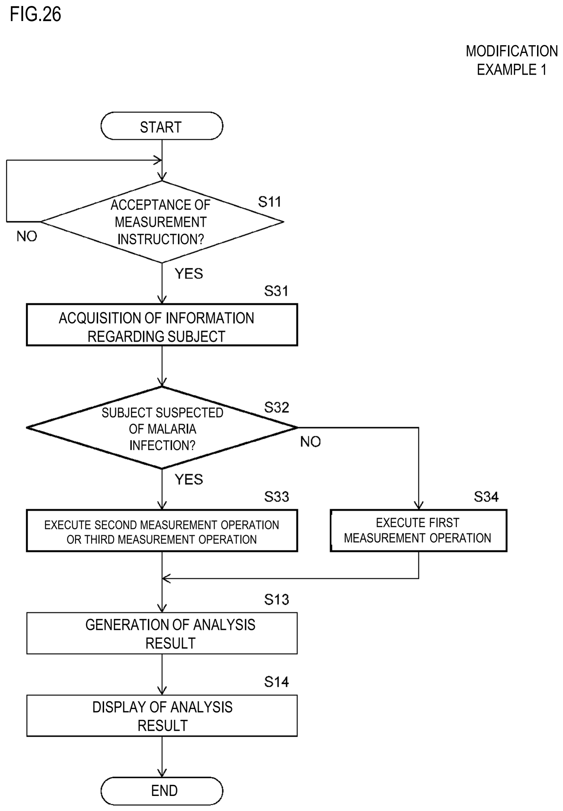

FIG. 26 is a flowchart showing a process performed by the controller 401 of the analysis unit 30 when a measurement operation is selectively executed based on information regarding a subject.

In the flowchart of FIG. 26, steps S31 to S34 are added instead of step S12 as compared to the first embodiment of FIG. 23.

In step S31, the controller 401 obtains other information regarding a subject from whom a measurement target sample has been collected from, for example, an electronic medical record system via a computer network. In step S32, the controller 401 determines whether or not the subject is a subject suspected of being infected with malaria, based on the other information regarding the subject obtained in step S31. For example, when information obtained from the electronic medical record system includes information indicating that the subject has a history of traveling to a malaria-endemic area, the controller 401 determines that the subject is a subject suspected of being infected with malaria.

When malaria infection is suspected (step S32: YES), the controller 401 controls the measurement unit 10 to execute a second measurement operation or a third measurement operation. When malaria infection is not suspected (step S32: NO), the controller 401 controls the measurement unit 10 to execute a first measurement operation.

Note that which of the second measurement operation and the third measurement operation is performed in step S33 may be set in advance by an operator or the like. A malaria mode discrete executed when the second measurement operation is performed and a multi-mode discrete executed when the third measurement operation is performed may be set in advance by an operator or the like.

### "<Effects of Sample Measurement Apparatus According to Modification Example 1>

The analysis unit 30 selects a measurement operation to be executed by the measurement unit 10 according to other information regarding a subject (steps S31 to S34 of FIG. 26)."

According to this configuration, since the first, second, and third measurement operations are automatically selected according to other information regarding a subject, it is possible to save the trouble of an operator selecting a measurement operation by referring to the other information regarding the subject.

When a sample is a sample obtained from a subject suspected of being infected with malaria (step S32: YES in FIG. 26), the analysis unit 30 selects a second measurement operation or a third measurement operation (step S33).

For a subject suspected of being infected with malaria, it is desirable to perform the second measurement operation or the third measurement operation including the measurement of the measurement sample M. According to the above configuration, since the second measurement operation or the third measurement operation is automatically selected for a sample obtained from a subject suspected of being infected with malaria, the trouble of judging a necessary measurement operation can be saved.

When a sample is a sample obtained from a subject not suspected of being infected with malaria (step S32: NO in FIG. 26), the analysis unit 30 selects a first measurement operation (step S34).

For a subject not suspected of being infected with malaria, the second measurement operation and the third measurement operation including the measurement of the measurement sample M are unnecessary. According to the above configuration, since the first measurement operation is automatically selected for a sample obtained from a subject not suspected of being infected with malaria, it is possible to prevent a situation in which an unnecessary measurement operation is selected.

### "<Modification Example 2>

In the first embodiment, in the multi-mode discrete, a measurement sample WDF and a measurement sample M are separately prepared, and measurements based on the measurement sample WDF and the measurement sample M are separately performed. However, the present invention is not limited to this, and one measurement sample WDF-M1 corresponding to both the measurement sample WDF and the measurement sample M may be prepared, and an analysis result similar to an analysis result based on the measurement sample WDF and the measurement sample M may be obtained based on the measurement of the measurement sample WDF-M1."

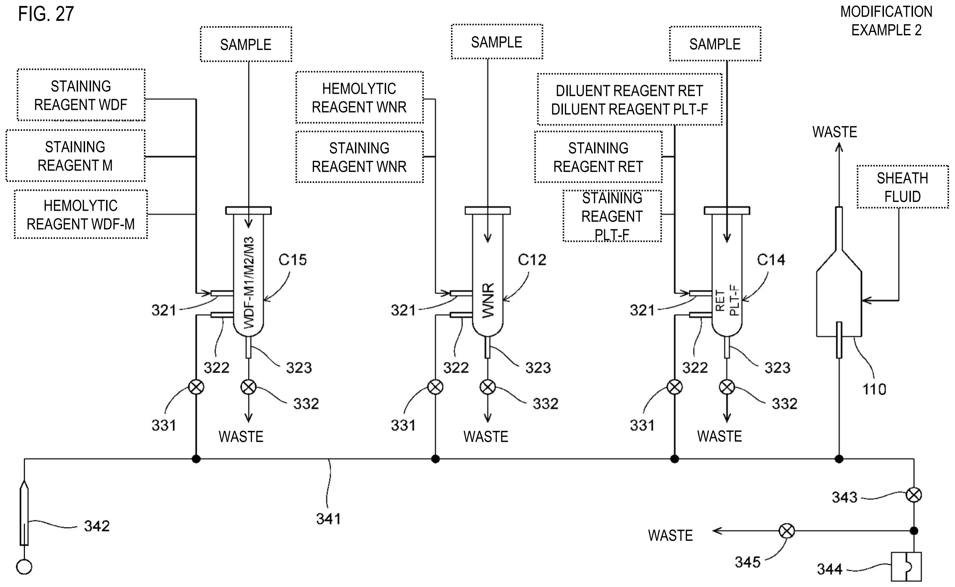

FIG. 27 is a diagram showing a configuration of a fluid circuit connected to chambers C12, C14, and C15 and the optical detector 110.

In this modification example, a chamber C15 is added instead of the chambers C11 and C13 as compared with the first embodiment of FIG. 6. The chamber C15 has the same configuration as the other chambers C12 and C14, and is connected to a flow path 341 similarly to the other chambers C12 and C14. Further, a staining reagent WDF, a staining reagent M, and a hemolytic reagent WDF-M are supplied through an inlet 321 of the chamber C15.

In this modification example, when the multi-mode discrete CBC+DIFF+MI and CBC+DIFF+RET+MI are selected by a measurement order, instead of preparing the measurement sample WDF and the measurement sample M, a sample, the staining reagent WDF, the staining reagent M, and the hemolytic reagent WDF-M are mixed in the chamber C15 to prepare a measurement sample WDF-M1. The hemolytic reagent WDF-M is a reagent that partially dissolves a cell membrane of red blood cells so that a fluorescent dye included in the staining reagent WDF and a fluorescent dye included in the staining reagent M can pass through while keeping malaria parasites inside the red blood cells. It is preferable that the dissolving power of the hemolytic reagent WDF-M to the cell membrane of red blood cells is stronger than that of the hemolytic reagent M and weaker than that of the hemolytic reagent WDF. The hemolytic reagent WDF-M includes, for example, a nonionic surfactant, a cationic surfactant, an anionic surfactant, an amphipathic surfactant, or a surfactant of a combination thereof.

When the preparation of the measurement sample WDF-M1 is completed, the measurement sample WDF-M1 is supplied to the optical detector 110 via the flow path 341, and measurement is performed in the optical detector 110 using the light sources 201 and 202.

When the measurement sample WDF-M1 is measured, a scattergram WDF in an upper stage of FIG. 16 and a scattergram M in an upper stage of FIG. 17 are generated based on a measurement result of the measurement sample WDF-M1. Then, the controller 401 classifies blood cells plotted on the scattergram WDF into any of (B11) to (B14), acquires the number of blood cells for each area, classifies blood cells plotted on the scattergram M into any of (B30), (B31), and (B36) or any of (B31) to (B36), and acquires the number of blood cells for each area.

When a normal mode discrete is selected, a measurement sample WDF-M2 is prepared by mixing a sample, the hemolytic reagent WDF-M, and the staining reagent WDF in the chamber C15, and the scattergram WDF in the upper stage of FIG. 16 is generated based on a measurement result of the measurement sample WDF-M2. When a malaria mode discrete is selected, a measurement sample WDF-M3 is prepared by mixing a sample, the hemolytic reagent WDF-M, and the staining reagent M in the chamber C15, and the scattergram M in the upper stage of FIG. 17 is generated based on a measurement result of the measurement sample WDF-M3. That is, in this modification example, the measurement sample WDF-M1, the measurement sample WDF-M2, and the measurement sample WDF-M3 are prepared in the common chamber C15.

### "<Effects of Sample Measurement Apparatus According to Modification Example 2>

In the third measurement operation, the measurement unit 10 prepares a common measurement sample WDF-M1 for classification of leukocytes and counting of cells suspected of malaria infection, and detects optical signals corresponding to cells in the measurement sample WDF-M1."

According to this configuration, in the third measurement operation, since only one measurement sample needs to be prepared for a leukocyte test and a malaria infection test, the third measurement operation can be performed more efficiently as compared to a case where a measurement sample WDF for a leukocyte test and a measurement sample M for a malaria infection test are separately prepared.

Further, according to this configuration, since measurement samples WDF-M1/M2/M3 can be prepared using the common hemolytic reagent WDF-M in the first measurement operation, the second measurement operation, and the third measurement operation, the number of reagents connected to the sample measurement apparatus 1 can be suppressed.

In the modification example 2, a reflex measurement may be performed as in the second embodiment. In this case, the reflex rule may include, for example, executing a multi-mode discrete when a flag of malaria-infected red blood cells is added to an analysis result of a normal mode. As a result, since an analysis result based on the measurement sample WDF-M1 can be further obtained, it is possible to more accurately determine whether or not a subject is suffering from malaria.

Further, in the modification example 2, when the multi-mode discrete is selected by a measurement order, the controller 401 performs an analysis based on the measurement sample WDF-M1, but the present invention is not limited to this, and the controller 401 may accept in advance from an operator which of a first analysis based on the measurement samples WDF-M2 and WDF-M3 and a second analysis based on the measurement sample WDF-M1 is to be performed. This makes it possible to determine which of the first analysis and the second analysis is to be performed, for example, according to an operational policy of a facility.

Further, in the modification example 2, the common chamber C15 for preparing the measurement sample WDF-M1, the measurement sample WDF-M2, and the measurement sample WDF-M3 is provided, but the present invention is not limited to this, and a chamber for preparing at least one of the measurement sample WDF-M1, the measurement sample WDF-M2, and the measurement sample WDF-M3 may be provided separately from the chamber C15.

### "<Modification Example 3>

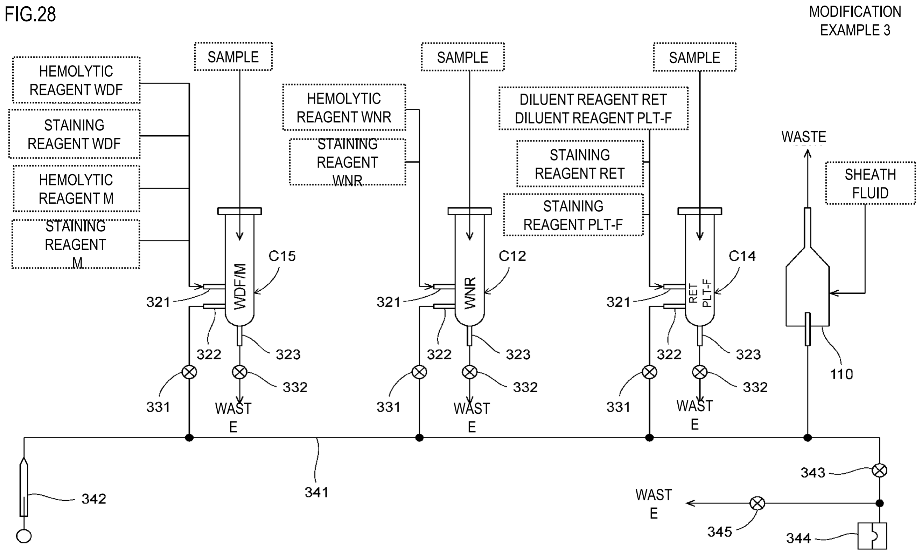

In the modification example 2, the staining reagent WDF, the staining reagent M, and the hemolytic reagent WDF-M are connected to the chamber C15. However, the present invention is not limited to this, and as shown in FIG. 28, the hemolytic reagent WDF, the staining reagent WDF, the hemolytic reagent M, and the staining reagent M may be connected to the chamber C15."

In this modification example, when a multi-mode discrete is selected, a measurement sample WDF is prepared by mixing a sample, the hemolytic reagent WDF, and the staining reagent WDF in the chamber C15, and the scattergram WDF in the upper stage of FIG. 16 is generated based on a measurement result of the measurement sample WDF. After the measurement sample WDF is discharged from the chamber C15 and the chamber C15 is washed, a measurement sample M is prepared by mixing a sample, the hemolytic reagent M, and the staining reagent M in the chamber C15, and the scattergram M in the upper stage of FIG. 17 is generated based on a measurement result of the measurement sample M. The preparation of the measurement sample WDF and the measurement sample M may be performed in any order.

When a normal mode discrete is selected, a measurement sample WDF is prepared by mixing a sample, the hemolytic reagent WDF, and the staining reagent WDF in the chamber C15, and the scattergram WDF in the upper stage of FIG. 16 is generated based on a measurement result of the measurement sample WDF. When a malaria mode discrete is selected, a measurement sample M is prepared by mixing a sample, the hemolytic reagent M, and the staining reagent M in the chamber C15, and the scattergram M in the upper stage of FIG. 17 is generated based on a measurement result of the measurement sample M. That is, in this modification example, the measurement sample WDF and the measurement sample M are prepared in the common chamber C15.

In this modification example, a reflex measurement may be performed as in the second embodiment.

### "<Modification Example 4>

In the first embodiment, the sample measurement apparatus 1 prepares a measurement sample WDF and a measurement sample WNR and creates a scattergram WDF and a scattergram WNR in measurement of a sub-discrete DIFF. In this modification example, the sample measurement apparatus 1 does not perform preparation of a measurement sample WNR and creation of a scattergram WNR, but prepares a measurement sample WDF and creates a scattergram WDF in measurement of a sub-discrete DIFF."

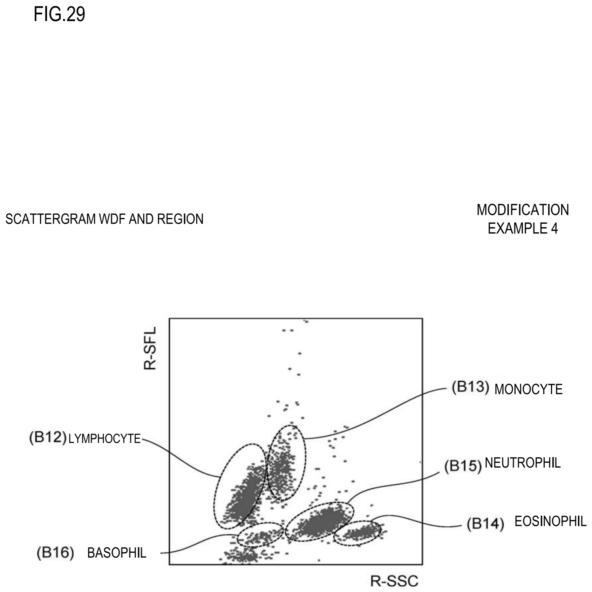

FIG. 29 shows an example of a scattergram WDF. The scattergram WDF is created based on a measurement result of the measurement sample WDF, similarly to the first embodiment.

The controller 401 sets five initial areas corresponding to lymphocytes, monocytes, eosinophils, neutrophils, and basophils for the scattergram WDF of FIG. 29 and performs a classification step described in the first embodiment. As a result, the controller 401 sets areas of (B15) neutrophils and (B16) basophils instead of (B11) neutrophils and basophils, as compared with the scattergram WDF in the upper stage of FIG. 16. Then, the controller 401 obtains the number of blood cells for each of the areas of (B12) to (B14), (B15), and (B16). Further, the controller 401 obtains the total number of blood cells of (B12) to (B14), (B15), and (B16) as a leukocyte count. That is, in this modification example, the measurement unit 10 prepares a measurement sample WDF and detects optical signals for cells in the measurement sample WDF in order to count and classify leukocytes.

### "<Effects of Sample Measurement Apparatus According to Modification Example 4>

The measurement unit 10 measures a sample for counting and classifying leukocytes in a first measurement operation."

According to this configuration, since only one measurement sample needs to be prepared for counting and classifying leukocytes, a measurement operation for counting and classifying leukocytes can be performed more efficiently as compared to a case where the measurement sample WDF and the measurement sample WNR are separately prepared.

### "<Modification Example 5>

In the first embodiment, a vertical axis and a horizontal axis of scattergrams WDF, WNR, M, RET, and PLT-F are set as shown in FIGS. 16 to 18. However, the present invention is not limited to this, and any one of optical signals based on light from the light sources 201 and 202 may be used as an optical signal used for an axis of each scattergram. In any case, an area corresponding to a type of axis is set so that blood cells can be appropriately classified for each scattergram."

However, when an optical signal based on fluorescence generated by light from the light source 201 is used, a staining reagent to be mixed with a measurement sample includes a fluorescent dye that can be excited by light of a wavelength λ10, and when an optical signal based on fluorescence generated by light from the light source 202 is used, a staining reagent to be mixed with a measurement sample includes a fluorescent dye that can be excited by light of a wavelength λ20.

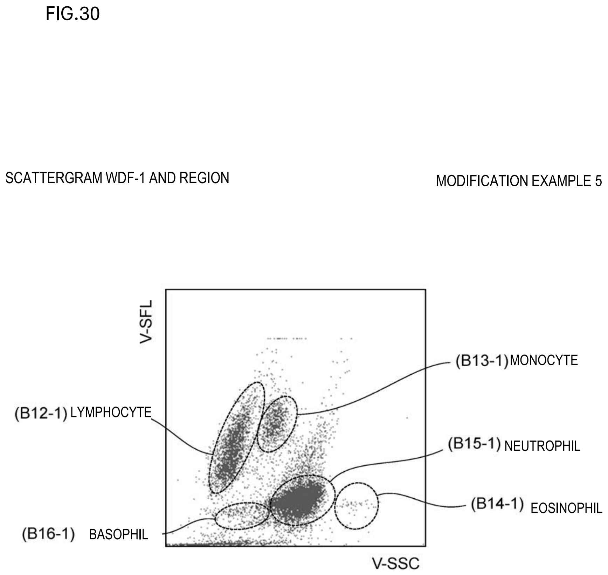

FIG. 30 shows an example of a scattergram WDF-1. The scattergram WDF-1 is created based on a measurement result of the measurement sample WDF. In the scattergram WDF-1, a horizontal axis corresponds to a level (V-SSC) of an optical signal detected based on side-scattered light generated by light of a wavelength λ10 (blue-violet wavelength band) emitted from the light source 201, and a vertical axis corresponds to a level (V-SFL) of an optical signal detected based on fluorescence generated by the light of the wavelength λ10 (blue-violet wavelength band) emitted from the light source 201.

The controller 401 sets five initial areas corresponding to lymphocytes, monocytes, eosinophils, neutrophils, and basophils for the scattergram WDF-1 and performs a classification step described in the first embodiment. As a result, the controller 401 sets areas of (B12-1) lymphocytes, (B13-1) monocytes, (B14-1) eosinophils, (B15-1) neutrophils, and (B16-1) basophils. Then, the controller 401 obtains the number of blood cells for each of the areas of (B12-1) to (B14-1), (B15-1), and (B16-1). Further, the controller 401 obtains the total number of blood cells of (B12-1) to (B14-1), (B15-1), and (B16-1) as a leukocyte count. That is, in this modification example, the measurement unit 10 prepares a measurement sample WDF and detects optical signals for cells in the measurement sample WDF in order to count and classify leukocytes.

### "<Effects of Sample Measurement Apparatus According to Modification Example 5>

The measurement unit 10 measures a sample for counting and classifying leukocytes in a first measurement operation."

According to this configuration, since only one measurement sample needs to be prepared for counting and classifying leukocytes, a measurement operation for counting and classifying leukocytes can be performed more efficiently as compared to a case where the measurement sample WDF and the measurement sample WNR are separately prepared.

In the first embodiment, only a light detector 221 for detecting forward-scattered light based on light of the wavelength λ20 is arranged as a light detector corresponding to the forward-scattered light. However, when forward-scattered light based on light of the wavelength λ10 is used for analysis, a light detector for detecting the forward-scattered light based on the light of the wavelength λ10 may be arranged instead of the light detector 221 or together with the light detector 221.

Further, when only an optical signal based on light of the wavelength λ10 from the light source 201 is used as an optical signal used for an axis of each scattergram, a light detector for detecting forward-scattered light based on the light of the wavelength λ10 is arranged instead of the light detector 221, and the light source 202, a dichroic mirror 241, and light detectors 242 and 243 are omitted.

### "<Modification Example 6>

In the first embodiment, when a discrete including a sub-discrete MI is selected, the measurement unit 10 measures a measurement sample RBC/PLT with an electrical detector 120 and measures a measurement sample M with an optical detector 110. However, the present invention is not limited to this, and the measurement unit 10 may measure the measurement sample M with the optical detector 110 without using the electrical detector 120 in a measurement operation (second measurement operation) for measuring the measurement sample M. In this case, the controller 401 calculates a total of the number of blood cells in areas of (B30) and (B31) (see the upper stage of FIG. 17) of the scattergram M as a red blood cell count necessary for calculating a measurement item MI-RBC%."

### "<Effects of Sample Measurement Apparatus According to Modification Example 6>

The measurement unit 10 includes an optical detector 110 configured to detect optical signals corresponding to cells in a measurement sample and an electrical detector 120 configured to detect electrical signals corresponding to the cells in the measurement sample. The measurement unit 10 does not use the electrical detector 120 in the second measurement operation."

According to this configuration, by not using the electrical detector 120 in the second measurement operation, the amount of use of a sample and a reagent can be suppressed.

### "<Other Modification Examples>

In the above-described embodiments and modification examples, when a measurement mode is selected, only a discrete corresponding to the measurement mode is displayed in a discrete selection area 612 of FIG. 12, and an actually executed discrete is determined when an operator selects a discrete in the discrete selection area 612. However, the present invention is not limited to this, and an actually executed discrete may be uniquely determined in accordance with the selection of the measurement mode. For example, when there are three types of discretes provided in the sample measurement apparatus 1, namely, CBC+DIFF, MI, and CBC+DIFF+MI, when a normal mode, a malaria mode, and a multi-mode are selected, CBC+DIFF, MI, and CBC+DIFF+MI may be determined as the discretes, respectively."

In the above-described embodiments and modification examples, leukocytes are classified into five subpopulations in measurement and analysis corresponding to a sub-discrete DIFF, but leukocytes may be classified into two subpopulations, three subpopulations, or four subpopulations.

In the above-described embodiments and modification examples, a predetermined amount of a measurement sample is measured in both of the first measurement operation and the second measurement operation, but the present invention is not limited to this, and for example, in measurement of a measurement sample M included in the second measurement operation, measurement may be continued until a predetermined number of malaria-infected red blood cells are detected. That is, a measurement time of the second measurement operation may be variable. This makes it possible to detect malaria-infected red blood cells more reliably. Further, in the measurement of the measurement sample M included in the second measurement operation, light output power of the light sources 201 and 202 may be increased as compared with the first measurement operation. In this case, detection sensitivity of malaria-infected red blood cells can be enhanced.

In the modification example 2, in the analysis result display screen 700 regarding the multi-mode shown in FIG. 22, when "malaria positive" is displayed in the flag display area 731, a message of "separate measurement of normal mode and malaria mode is required" and a checkbox may be displayed in the measurement necessity setting area 732. In this case, when an operator operates the checkbox and sets it to a checked state, a flag indicating that it is preferable to perform measurement regarding leukocyte classification (for example, measurement of a discrete CBC+DIFF) and measurement regarding malaria (for example, measurement of a discrete MI) may be added to an analysis result.

In the above-described embodiments and modification examples, when the measurement necessity setting area 732 shown in FIGS. 20 and 21 is set to a checked state, a measurement order regarding a necessary measurement operation may be automatically registered.

In the above-described embodiments and modification examples, a measurement order is stored in the storage 402 of the analysis unit 30, but may be stored in a host computer capable of communicating with the analysis unit 30. In this case, when a measurement order is input to the sample measurement apparatus 1 or another apparatus, the input measurement order is transmitted to the host computer and is centrally managed in the host computer. When the analysis unit 30 receives a sample ID read by the reader 161, the analysis unit 30 queries the host computer for a measurement order and receives a measurement order corresponding to the sample ID from the host computer.

Embodiments of the present invention can be appropriately modified in various ways within the scope of the technical idea shown in the claims.

### [Remarks]

The present disclosure includes following items 1-28
Item 1. A sample measurement apparatus configured to measure a sample collected from a subject, comprising:
   a measurement unit configured to execute a measurement of the sample, the measurement including preparing a measurement sample from the sample and a reagent and detecting at least optical signals corresponding to cells in the measurement sample; and
   an analysis unit configured to analyze the cells at least according to the optical signals detected in the measurement of the sample by the measurement unit,
   wherein the measurement unit is operable to selectively perform a plurality of measurement operations including:
      (1) a first measurement operation that includes the measurement of the sample using a first reagent containing a first fluorescent dye that stains leukocytes for classification of leukocytes;
      (2) a second measurement operation that includes the measurement of the sample using a second reagent containing a second fluorescent dye that stains cells suspected of malaria infection; and
      (3) a third measurement operation that includes the measurement of the sample using the first reagent and the second reagent.
Item 2. The sample measurement apparatus according to item 1, wherein
   the measurement unit includes a plurality of mechanisms configured to measure the measurement sample, and
   the measurement unit is configured to share at least one of the plurality of mechanisms among the first measurement operation, the second measurement operation, and the third measurement operation.
Item 3. The sample measurement apparatus according to item 1, wherein
   the measurement unit includes an optical detector configured to detect the optical signals corresponding to the cells in the measurement sample, and
   the measurement unit is configured to share the optical detector among the first measurement operation, the second measurement operation, and the third measurement operation.
Item 4. The sample measurement apparatus according to item 1, wherein
   the measurement unit includes at least one light source configured to irradiate the measurement sample with light, and
   the measurement unit is configured to share the at least one light source among the first measurement operation, the second measurement operation, and the third measurement operation.
Item 5. The sample measurement apparatus according to item 1, wherein
   the measurement unit includes a first light source configured to irradiate the measurement sample with light of a first wavelength and a second light source configured to irradiate the measurement sample with light of a second wavelength, and
   the measurement unit is configured to irradiate the measurement sample with at least one of the light of the first wavelength and the light of the second wavelength in the first measurement operation, and to irradiate the measurement sample with the light of the first wavelength and the light of the second wavelength in the second measurement operation.
Item 6. The sample measurement apparatus according to item 5, wherein
   the first wavelength is 315 nm or more and 490 nm or less, and
   the second wavelength is 610 nm or more and 750 nm or less.
Item 7. The sample measurement apparatus according to item 1, wherein
   the measurement unit includes an aspiration tube configured to aspirate the sample provided to the sample measurement apparatus, and
   the measurement unit is configured to share the aspiration tube among the first measurement operation, the second measurement operation, and the third measurement operation.
Item 8. The sample measurement apparatus according to item 1, wherein
   the measurement unit includes an aspiration tube configured to aspirate the sample provided to the sample measurement apparatus and a pump configured to aspirate the sample in the aspiration tube, and
   the measurement unit is configured to share the aspiration tube and the pump among the first measurement operation, the second measurement operation, and the third measurement operation.
Item 9. The sample measurement apparatus according to item 1, wherein
   the measurement unit is configured to execute the first measurement operation and the second measurement operation in the third measurement operation.
Item 10. The sample measurement apparatus according to item 1, wherein
   the measurement unit includes a plurality of mechanisms configured to measure the measurement sample, and
   at least one of the plurality of mechanisms is configured to perform different operations in each of the first measurement operation and the second measurement operation.
Item 11. The sample measurement apparatus according to item 1, wherein
   the measurement unit includes an optical detector configured to detect the optical signals corresponding to the cells in the measurement sample, and
   a measurement time by the optical detector in the first measurement operation is different from a measurement time by the optical detector in the second measurement operation.
Item 12. The sample measurement apparatus according to item 1, wherein
   the measurement unit includes a sample preparation unit configured to prepare the measurement sample from the sample and the reagent, and
   the sample preparation unit is configured to prepare a first measurement sample from the sample and the first reagent in the first measurement operation, and to prepare a second measurement sample from the sample and the second reagent in the second measurement operation.
Item 13. The sample measurement apparatus according to item 12, wherein
   the sample preparation unit is configured to prepare the first measurement sample from the sample, the first reagent, and a first hemolytic reagent in the first measurement operation, and to prepare the second measurement sample from the sample, the second reagent, and a second hemolytic reagent in the second measurement operation.
Item 14. The sample measurement apparatus according to item 1, wherein
   the measurement unit is configured to measure the sample for counting and classifying the leukocytes in the first measurement operation.
Item 15. The sample measurement apparatus according to item 1, wherein
   the measurement unit is configured to measure the sample for counting the cells suspected of malaria infection and counting the leukocytes in the second measurement operation.
Item 16. The sample measurement apparatus according to item 1, wherein
   the measurement unit is configured to prepare a common measurement sample for classification of the leukocytes and counting of the cells suspected of malaria infection in the third measurement operation, and to detect the optical signals corresponding to the cells in the common measurement sample.
Item 17. The sample measurement apparatus according to item 1, wherein
   the measurement unit includes an optical detector configured to detect the optical signals corresponding to the cells in the measurement sample and an electrical detector configured to detect electrical signals corresponding to the cells in the measurement sample, and
   the measurement unit is configured to use both the optical detector and the electrical detector in the first measurement operation and the second measurement operation.
Item 18. The sample measurement apparatus according to item 1, wherein
   the measurement unit includes an optical detector configured to detect the optical signals corresponding to the cells in the measurement sample and an electrical detector configured to detect electrical signals corresponding to the cells in the measurement sample, and
   the measurement unit is configured not to use the electrical detector in the second measurement operation.
Item 19. The sample measurement apparatus according to item 1, wherein
   the analysis unit is configured to analyze the cells by different clustering in the first measurement operation and the second measurement operation.
Item 20. The sample measurement apparatus according to item 1, wherein
   the analysis unit is configured to classify the leukocytes into a plurality of subpopulations in analysis corresponding to the first measurement operation.
Item 21. The sample measurement apparatus according to item 1, wherein
   the analysis unit is configured to obtain information regarding a malaria life cycle for the cells suspected of malaria infection in analysis corresponding to the second measurement operation.
Item 22. The sample measurement apparatus according to item 1, wherein
   the analysis unit is configured to select the measurement operation to be executed by the measurement unit according to information regarding the sample.
Item 23. The sample measurement apparatus according to item 22, wherein
   the analysis unit is configured to select the second measurement operation or the third measurement operation when the sample is obtained from a subject suspected of being infected with malaria.
Item 24. The sample measurement apparatus according to item 22, wherein
   the analysis unit is configured to select the first measurement operation when the sample is obtained from a subject not suspected of being infected with malaria.
Item 25. The sample measurement apparatus according to item 1, wherein
   the analysis unit includes a controller, a display, and an operation unit, and
   the controller is programmed to:
      cause the display to display a screen for selecting one measurement mode from three measurement modes, the three measurement modes classifying the plurality of measurement operations respectively to the first measurement operation, the second measurement operation, and the third measurement operation, and
      accept the measurement mode selected via the screen by operation of the operation unit, and
      causes the display to display another screen for selecting the measurement operation corresponding to the accepted measurement mode.
Item 26. The sample measurement apparatus according to item 1, wherein
   the analysis unit includes a controller, a display, and an operation unit, and
   the controller is programmed to:
      cause the display to display a screen for selecting the first measurement operation, the second measurement operation, or the third measurement operation from the plurality of measurement operations, and
   accept the measurement operation selected via the screen by operation of the operation unit.
Item 27. The sample measurement apparatus according to item 1, wherein
   the analysis unit includes a controller, a display, and an operation unit, and
   the controller is programmed to:
      cause the display to display a screen for selecting at least one measurement item from a plurality of measurement items that respectively define the first measurement operation, the second measurement operation, and the third measurement operation, and
      accept the measurement item selected via the screen by operation of the operation unit.
Item 28. The sample measurement apparatus according to item 1, wherein
   the analysis unit includes a controller, a display, and an operation unit, and
   the controller is programmed to:
      cause the display to display a screen for registering a measurement order indicating which of the first measurement operation, the second measurement operation, and the third measurement operation is to be executed, and
      accept the measurement order registered via the screen by operation of the operation unit.

## Claims

1. A sample measurement apparatus configured to measure a sample collected from a subject, comprising:
a measurement unit configured to execute a measurement of the sample, the measurement including preparing a measurement sample from the sample and a reagent and detecting at least optical signals corresponding to cells in the measurement sample; and
an analysis unit configured to analyze the cells at least according to the optical signals detected in the measurement of the sample by the measurement unit,
wherein the measurement unit is operable to selectively perform a plurality of measurement operations including:
(1) a first measurement operation that includes the measurement of the sample using a first reagent containing a first fluorescent dye that stains leukocytes for classification of leukocytes;
(2) a second measurement operation that includes the measurement of the sample using a second reagent containing a second fluorescent dye that stains cells suspected of malaria infection; and
(3) a third measurement operation that includes the measurement of the sample using the first reagent and the second reagent.

2. The sample measurement apparatus according to claim 1, wherein
the measurement unit includes a plurality of mechanisms configured to measure the measurement sample, and
the measurement unit is configured to share at least one of the plurality of mechanisms among the first measurement operation, the second measurement operation, and the third measurement operation.

3. The sample measurement apparatus according to claim 1 or 2, wherein
the measurement unit includes an optical detector configured to detect the optical signals corresponding to the cells in the measurement sample, and
the measurement unit is configured to share the optical detector among the first measurement operation, the second measurement operation, and the third measurement operation.

4. The sample measurement apparatus according to any one of claims 1 to 3, wherein
the measurement unit includes a first light source configured to irradiate the measurement sample with light of a first wavelength and a second light source configured to irradiate the measurement sample with light of a second wavelength, and
the measurement unit is configured to irradiate the measurement sample with at least one of the light of the first wavelength and the light of the second wavelength in the first measurement operation, and to irradiate the measurement sample with the light of the first wavelength and the light of the second wavelength in the second measurement operation.

5. The sample measurement apparatus according to any one of claims 1 to 4, wherein
the measurement unit includes an aspiration tube configured to aspirate the sample provided to the sample measurement apparatus and a pump configured to aspirate the sample in the aspiration tube, and
the measurement unit is configured to share the aspiration tube and the pump among the first measurement operation, the second measurement operation, and the third measurement operation.

6. The sample measurement apparatus according to any one of claims 1 to 5, wherein
the measurement unit is configured to execute the first measurement operation and the second measurement operation in the third measurement operation.

7. The sample measurement apparatus according to any one of claims 1 to 6, wherein
the measurement unit includes a plurality of mechanisms configured to measure the measurement sample, and
at least one of the plurality of mechanisms is configured to perform different operations in each of the first measurement operation and the second measurement operation.

8. The sample measurement apparatus according to any one of claims 1 to 7, wherein
the measurement unit includes an optical detector configured to detect the optical signals corresponding to the cells in the measurement sample, and
a measurement time by the optical detector in the first measurement operation is different from a measurement time by the optical detector in the second measurement operation.

9. The sample measurement apparatus according to any one of claims 1 to 8, wherein
the measurement unit includes a sample preparation unit configured to prepare the measurement sample from the sample and the reagent, and
the sample preparation unit is configured to prepare a first measurement sample from the sample and the first reagent in the first measurement operation, and to prepare a second measurement sample from the sample and the second reagent in the second measurement operation.

10. The sample measurement apparatus according to any one of claims 1 to 9, wherein
the measurement unit is configured to measure the sample for counting and classifying the leukocytes in the first measurement operation.

11. The sample measurement apparatus according to any one of claims 1 to 10, wherein
the measurement unit is configured to measure the sample for counting the cells suspected of malaria infection and counting the leukocytes in the second measurement operation.

12. The sample measurement apparatus according to any one of claims 1 to 11, wherein
the measurement unit is configured to prepare a common measurement sample for classification of the leukocytes and counting of the cells suspected of malaria infection in the third measurement operation, and to detect the optical signals corresponding to the cells in the common measurement sample.

13. The sample measurement apparatus according to any one of claims 1 to 12, wherein
the measurement unit includes an optical detector configured to detect the optical signals corresponding to the cells in the measurement sample and an electrical detector configured to detect electrical signals corresponding to the cells in the measurement sample, and
the measurement unit is configured to use both the optical detector and the electrical detector in the first measurement operation and the second measurement operation.

14. The sample measurement apparatus according to any one of claims 1 to 13, wherein
the analysis unit is configured to analyze the cells by different clustering in the first measurement operation and the second measurement operation.

15. The sample measurement apparatus according to any one of claims 1 to 14, wherein
the analysis unit is configured to select the measurement operation to be executed by the measurement unit according to information regarding the sample.
